# EUROPEAN PATENT SPECIFICATION

(11) **EP 2 387 717 B1**
(45) Date of publication and mention of the grant of the patent: **10.12.2014**
(21) Application number: 10732178.8
(22) Date of filing: 15.01.2010
(51) Int. Cl.: G01N 33/48, G01N 33/53, G01N 33/574, C07K 16/28

(54) **METHODS OF DETERMINING PATIENT RESPONSE BY MEASUREMENT OF HER-2 EXPRESSION**
VERFAHREN ZUR BESTIMMUNG VON PATIENTENREAKTIONEN DURCH MESSUNG DER HER-2-EXPRESSION
PROCÉDÉS DE DÉTERMINATION D'UNE RÉPONSE DE PATIENT PAR LA MESURE DE L'EXPRESSION DE HER-2

(30) Priority: 15.01.2009 US 145029 P
(43) Date of publication of application: 23.11.2011
(73) Proprietor: Laboratory Corporation of America Holdings, Burlington, NC 27215 (US)
(72) Inventor: BATES, Michael, San Carlos, California 94070 (US); COOK, Jennifer, W., San Mateo, California 94403 (US); DIEDRICH, Gundo, South San Franciso California 94080 (US); GOODMAN, Laurie, El Granada, California 94018 (US); MUKHERJEE, Ali, Millbrae, California 94030 (US); PARRY, Gordon, Oakland, California 94619 (US); SPERINDE, Jeff, El Granada, California 94018-2045 (US); WILLIAMS, Stephen John, San Carlos, California 94070 (US)
(74) Representative: Donald, Jenny Susan
(86) International application number: PCT/US2010/021272
(87) International publication number: WO 2010/083463

(56) References cited:
- WO-A2-2006/044748
- US-A1- 2007 037 228
- US-A1- 2008 182 255
- US-A1- 2009 011 440
- MUKHERJEE ALI ET AL: "Correlation of ErbB activation status and clinical response in Herceptin treated breast cancer patients.", PROCEEDINGS OF THE AMERICAN ASSOCIATION FOR CANCER RESEARCH ANNUAL MEETING, vol. 46, April 2005 (2005-04), pages 869-870, XP008152496, & 96TH ANNUAL MEETING OF THE AMERICAN-ASSOCIATION-FOR-CANCER-RESEARCH; ANAHEIM, CA, USA; APRIL 16 -20, 2005 ISSN: 0197-016X
- BATES M P ET AL: "HER2 expression and HER2:HER2 dimerization identifiessubpopulations of metastatic breast cancer patients withdifferent probabilities of long-term survival followingtrastuzumab treatment and with different requirements forconcomitant chemotherapy", JOURNAL OF CLINICAL ONCOLOGY; 2007 ASCO ANNUAL MEETING [AMERICAN SOCIETY OF CLINICAL ONCOLOGY]; JUNE 1 - 5, 2007; CHICAGO, ILLINOIS, AMERICAN SOCIETY OF CLINICAL ONCOLOGY, US, vol. 25, no. 18S, 20 June 2007 (2007-06-20), pages 1-3, XP002612825, ISSN: 0732-183X
- HUANG W ET AL: "Quantitative measurements of HER2 expression and HER2: HER2 dimerization identify subgroups of HER2 positive metastatic breast cancer patients with different probabilities of response to trastuzumab treatment", BREAST CANCER RESEARCH AND TREATMENT; & 30TH ANNUAL SAN ANTONIO BREAST CANCER SYMPOSIUM; SAN ANTONIO, TX, USA; DECEMBER 13 -16, 2007, SPRINGER, NEW YORK, NY, vol. 106, no. Suppl. 1, 13 December 2007 (2007-12-13), page S86, XP002612828, ISSN: 0167-6806
- WINSLOW J ET AL: "Characterization of a novel proximity immunoassay for the quantitative determination of HER2 protein expression and HER2 homodimerization in formalin-fixed, paraffin-embedded breast cancer tissue", BREAST CANCER RESEARCH AND TREATMENT; & 30TH ANNUAL SAN ANTONIO BREAST CANCER SYMPOSIUM; SAN ANTONIO, TX, USA; DECEMBER 13 -16, 2007, SPRINGER, NEW YORK, NY, vol. 106, no. Suppl. 1, 13 December 2007 (2007-12-13), page S88, XP002612827, ISSN: 0167-6806
- SIAN LENNON, CLAIRE BARTON, LUDGER BANKEN, LUCA GIANNI, MICHEL MARTY, JOSE BASELGA, BRIAN LEYLAND-JONES: "Clinical Decision Making: Pooled Analysis of Four Trials of Trastuzumab in Metastatic Breast Cancer", JOURNAL OF CLINICAL ONCOLOGY, vol. 27, no. 10, 2 March 2009 (2009-03-02) , pages 1685-1693,

## Description

### Field of the Invention

The present invention provides methods for determining whether a cancer patient is likely to respond to treatment with a HER2-acting agent. The methods provide probable time to progression of a subclass of HER2 positive patients by further stratifying them using another biomarker, such as the presence or the absence of HER3 markers.

### Background of the Invention

Expression levels of individual cell surface receptors, such as Her-2, have been used as biomarkers. Conventional immunohistochemical (IHC) or fluorescence *in situ* hybridization (FISH) analysis has been used to detect Her-2 overexpression to determine whether treatment with a Her2-acting agent, e.g., trastuzumab, is warranted. Also, U.S. Patent 4,968,603 describes Her-2 expression as a cancer biomarker. However, in two different studies, only 20% or 35% of patients overexpressing Her-2 objectively responded to trastuzumab treatment. *See* Baselga et al., 1996, J. Clin. Oncol. 14:737-44; Cobleigh et al., 1999, J. Clin. Oncol. 17:2639-48; and Vogel et al., 2002, J. Clin. Oncol. 20:719-26. Further, in other studies of the combination of trastuzumab plus chemotherapy in the metastatic breast cancer setting, only approximately 50% of patients overexpressing Her-2 objectively responded to trastuzumab combination therapy. *See* Slamon et al. N Engl J Med 344: 783-92.

At the current time it can be problematic to determine whether a subject with a cancer is likely or unlikely to respond to treatment with a Her-2-acting agent, such as trastuzumab. Determining whether such patients are unlikely to respond to trastuzumab and/or other Heracting agents would avoid providing costly but ineffective treatment to those patients. For example, an assay to determine patient sensitivity to Her-2 acting agents may also be used to identify patients that are unlikely to respond to a chemotherapeutic agent in addition to the Her-2 acting agent thus allowing the subject to avoid the potentially toxic effects of the chemotherapeutic agent. Also, an assay to determine patient sensitivity to Her-2 acting agents may be used to predict a time course of disease or a probability of a significant event in the disease for Her-2 positive patients. Thus, there is a need for a method to determine whether a cancer patient will be responsive to Her-2 acting agents so as to maximize therapy for the patient.

### Summary of the Invention

The present invention provides methods for determining whether a subject with a cancer is likely to respond to treatment with a Her2-acting agent as specified in the appended claims. For example, in certain embodiments, the present disclosure comprises methods to correlate the relative levels of the amount of at least one of total Her-2 (H2T) or Her-2 homodimers in a biological sample from a subject with a prognosis for the likelihood that the subject will respond to treatment with a Her-2 acting agent comprising: (a) detecting in a biological sample from the subject's cancer the amount of at least one of Her-2 or Her-2 homodimers; and (b) correlating the amount of Her-2 or Her-2 homodimers to a prognosis for the likelihood that the subject will respond to treatment with a Her-2 acting agent.

In certain embodiments, the disclosure also provides methods for predicting a time course of disease and/or a probability of a significant event in the time course of disease in a subject with a cancer based on the predicted sensitivity of a patient to a Her2-acting agent. In certain embodiments, the methods comprise detecting a biomarker or combination of biomarkers associated with responsiveness to treatment with a Her2-acting agent as described hereinafter, and determining whether the subject is likely to respond to treatment with the Her2-acting agent. In certain embodiments, the methods comprise detecting a biomarker or combination of biomarkers and predicting a time course associated with progression of disease or a probability of a significant event in the time course of disease in a subject with cancer.

For example, in certain embodiments, the disclosure comprises methods for predicting whether a subject with a cancer and being treated with a Her-2 acting agent is likely to have a significant event comprising the steps of: (a) detecting in a biological sample from the subject's cancer the amount of at least one of Her-2 or Her-2 homodimers; and (b) correlating the amount of Her-2 or Her-2 homodimers to the likelihood that the subject will have a significant event.

In other aspects, the invention is drawn to a method for determining whether a subject with a cancer is likely to respond to treatment with a Her2-acting agent. In another aspect, the invention is drawn to a method for predicting a time course of disease. In another aspect, the method is drawn to a method for predicting a probability of a significant event in the time course of the disease.

In certain embodiments of each of the methods, the method comprises detecting in a biological sample from the subject's cancer the amount of Her-2 and/or Her-2 homodimers and determining if the Her-2 and/or Her-2 homodimers correlate to a low or high level of Her-2 expression.

In certain embodiments of each of the methods, high Her-2 expression is a log10H2T (log 10 of total Her-2) ≥ about 1.14-1.125. In certain embodiments of each of the methods disclosed herein, the high Her-2 expression comprises expression that is very high and/or moderately high. In certain embodiments of each of the methods disclosed herein, very high Her-2 expression is a log10H2T ≥ about 1.84 -2.21. In certain embodiments of each of the methods disclosed herein, moderately high Her-2 expression is between a log10H2T between about 1.14 - 1.25 and 1.84-2.21 (i.e., ≥ 1.14 - 1.25 and ≤ 1.84 - 2.21. Or, other ranges may be used depending upon the patient cohort and/or the significant event being monitored. Thus, each of the threshold values and/or threshold ranges described herein may vary by 0.5 log units when described on a log10 scale or by about 25% on a linear scale or less (i.e., be ≤ 25% larger and/or ≤25% smaller than the specific ranges disclosed herein), or by about 20% or less, or by about 15% or less, or by about 10% or less, or by about 5% or less.

In certain embodiments, if the amount of Her-2 and/or Her-2 homodimers is high, then the patient is likely to respond to the Her-2 acting agent and/or the patient has a long time course.

In some embodiments, if the amount of Her-2 and/or Her-2 homodimers is moderately high, then the patient is likely to respond to the Her-2 acting agent and/or the patient has a long time course.

Also, in some embodiments, if the amount of Her-2 and/or Her-2 homodimers is very high and/or low, then the patient is unlikely to respond to the Her-2 acting agent and/or the patient has a short time course.

Thus, in certain embodiments, if the amount of the at least one of the Her-2 or Her-2 homodimers is above a first threshold level (e.g., "high"), the subject's prognosis is to be likely to respond to the Her-2 acting agent. Additionally and/or alternatively, in certain embodiments and as discussed in more detail herein, if the amount of the at least one of the Her-2 or Her-2 homodimers is above a second threshold level higher than the first threshold level (e.g., "very high"), the subject's prognosis is to be unlikely to respond to the Her-2 acting agent.

In certain embodiments, the cancer is breast cancer. In some embodiments, the breast cancer is metastatic. In some embodiments, the breast cancer is early stage (i.e., adjuvant) breast cancer. In certain embodiments, the Her-2 acting agent is trastuzumab. In certain embodiments, the method is performed with an VERATAG® assay. In certain embodiments, likeliness to respond is measured with respect to overall survival rate, time to progression and/or using the RECIST or other response criteria.

In certain embodiments, a predetermined measure is created by dividing patient samples into at least three patient subgroups. For example, in certain embodiments, the patients are divided into a subgroup with low Her-2 expression (i.e., Her2 total and/or Her-2 dimers) and a subgroup with high Her-2 expression. The subgroup with high Her-2 expression may then be subdivided into a group having very high Her-2 and moderately high Her-2 expression. Thus, in certain embodiments, the number of subgroups is three so that the patient sample is divided into a subgroup of patients whose Her-2 and/or Her-2 homodimers is very high, a subgroup whose Her-2 and/or Her-2 homodimers is low, and a subgroup whose Her-2 and/or Her-2 homodimers is moderately high. In certain embodiments, the amount of Her-2 and/or Her-2 homodimers in the subject are compared to either the high subgroup or the low subgroup; if the amount of Her-2 and/or Her-2 homodimers in the patient are moderately high, then the patient is likely to respond to a Her-2 acting agent and/or the patient is likely to have a long time course.

For example, in certain embodiments of each of the methods disclosed herein, a predetermined measure is created by dividing a plurality of subject samples into at least three subgroups, wherein the first subgroup comprises samples having the Her-2 or Her-2 homodimers at a low level, wherein the low level comprises having an amount of the at least one of the Her-2 or Her-2 homodimers equal to or below a first threshold level, and the samples having an amount of the at least one of the Her-2 or Her-2 homodimers equal to or above the first threshold comprise samples having a high level of the Her-2 or Her-2 homodimers, and wherein the samples having a high level of the Her-2 or Her-2 homodimers is then divided into two subgroups, a very high subgroup comprising samples having an amount of the at least one of the Her-2 or Her-2 homodimers equal to or above a second threshold level higher than the first threshold level, and a moderately high subgroup comprising samples having an amount of the at least one of the Her-2 or Her-2 homodimers greater than or equal to the first threshold level and less than or equal to the second threshold level.

In each of the embodiments of the methods, where Her-2 is measured, the Her-3 expression may comprise Her-2, Her-2 homodimers, or Her-2 heterodimers. For example, in certain embodiments, the amount of total Her-2, Her-2 homodimers and/or Her-2 heterodimers is measured using an assay capable of measuring and/or quantifying an amount of protein-protein interactions in a sample.

In another embodiment, the subgroup whose Her-2 and/or Her-2 homodimers is moderately high (i.e., medium) is further subdivided into subgroups that express another biomarker. The other biomarker can be at least one of FOXM1, PRAME, Bc12, STK15, CEGP1, Ki-67, GSTM1, CA9, PR, BBC3, NME1, SURV, GATA3, TFRC, YB-1, DPYD, GSTM3, RPS6KB1, Src, Chk1, ID1, EstR1, p27, CCNB1, XIAP, Chk2, CDC25B, IGF1R, AK055699, P13KC2A, TGFB3, BAGI1, CYP3A4, EpCAM, VEGFC, pS2, hENT1, WISP1, HNF3A, NFKBp65, BRCA2, EGFR, TK1, VDR, Contig51037, pENT1, EPHX1, IF1A, CDH1, HIF1α, IGFBP3; CTSB, Her3 or DIABLO. In certain embodiments, the other biomarker can be VEGF, CD31, KDR, p95, or Her3. In other embodiments; the biomarker can be Her3. The additional marker may be used to further distinguish the Her-2 subgroups.

In certain embodiments, a predetermined measure is created by dividing Her-2 moderately high patient samples into at least two patient subgroups. In certain embodiments, the number of subgroups is two so that the patient sample is divided into a subgroup of patients whose Her-3 expressoion is high and another subgroup of patients whose Her3 expressoion is low.

Thus, in certain embodiments of each of the methods, wherein the moderately high subgroup is further divided based upon Her-3 expression wherein a high level comprises having Her-3 equal to or above a first threshold level and a low level comprises having Her-3 below the first threshold level, and wherein a subject with moderately high levels of the at least one Her-2 and/or Her-2 dimers and low levels of Her-3 is likely to respond to the Her-2 acting agent and/or wherein a subject with moderately high levels of the at least one Her-2 and/or Her-2 dimers and high levels of Her-3 is unlikely or less likely to respond to the Her-2 acting agent.

Where Her-3 is measured, the Her-3 expression may comprise Her-3, Her-3 homodimers, or Her-3 heterodimers. For example, in certain embodiments, the amount of total Her-3, Her-3 homodimers and/or Her-3 heterodimers (e.g., Her2/Her-3) is measured using an assay capable of measuring and/or quantifying an amount of protein-protein interactions in a sample.

For example, in one embodiment the cut-off for Her3 high expression (as compared to Her3 low expression is 0.158). Or, values about 25% lower and/or 25% higher may be used. Thus, each of the threshold values and/or threshold ranges described herein may vary by about 0.5 log units for a log10 scale and/or 25% on a linear scale or less (i.e., be ≤ 25% larger and/or ≤25% smaller than the specific ranges disclosed herein), or by about 20% or less, or by about 15% or less, or by about 10% or less, or by about 5% or less.

The actual value for a Her3 high vs. low cut-off may vary depending upont the patient cohort and/or the significant event being monitored. In certain embodiments, the number of subgroups is greater than three, including, without limitation, four subgroups, five subgroups and six subgroups. In certain embodiments, likeliness to respond is measured with respect to overall survival rate, time to progression and/or using the RECIST or other response criteria. In certain preferred embodiments, the Her-2 acting agent is trastuzumab.

In another aspect, the invention is drawn to a method for determining whether a subject with a Her-2 positive cancer is likely to respond to treatment with a Her2-acting agent and/or the time course of the disease is long. In another aspect, the invention is drawn to a method for predicting a time course of disease in a subject with a Her-2 positive cancer. In another aspect, the invention is drawn to a method for predicting the probability of a significant event in a subject with a Her-2 positive cancer.

Thus, in certain embodiments of each of the methods disclosed, the method comprises measuring in a biological sample from the subject's cancer an amount of Her-2 and/or Her-2 homodimers, wherein if the amount of Her-2 and/or Her-2 homodimers is moderately high and Her-3 expression is low, then the patient is likely to respond to the Her-2 acting agent and/or the patient has a long time course. In certain embodiments, the method comprises measuring in a biological sample from the subject's cancer an amount of Her-2 and/or Her-2 homodimers, wherein if the amount of Her-2 and/or Her-2 homodimers is moderately high and Her-3 expressionis high, then the patient is unlikely to respond to the Her-2 acting agent and/or the patient has a short time course. In certain embodiments, the biological sample comprises FFPEs. In certain embodiments, the subject's cancer is breast cancer. In certain embodiments, the breast cancer is metastatic. In certain embodiments, the breast cancer is early stage (i.e., adjuvant) breast cancer. In certain embodiments, the Her-2 acting agent is trastuzumab.

In certain embodiments, an amount of Her-2 is measured. In certain embodiments, an amount of Her-2 homodimers is measured. For example, in certain embodiments, the level of total Her-2 is correlated to the level of Her-2 homodimers such that measurement of either provides the same prognostic indications (i.e., whether a patient will respond to a Her-2 acting agent). In certain embodiments, the amount of Her-2 homodimers is measured using an assay capable of measuring and/or quantifying an amount of protein-protein interactions in a sample. In certain embodiments, the assay is the VERATAG® assay. In certain embodiments, likeliness to respond is measured with respect to overall survival rate, time to progression and/or using the RECIST or other response criteria.

In another aspect, the disclosure provides a method for determining whether a subject with Her2-positive cancer is unlikely to respond to treatment with a Her2-acting agent and/or the patient is likely to have a short time course. In certain embodiments, the method comprises detecting in a biological sample from the subject's cancer the amount of Her-2, wherein if the amount of Her-2 is low, the subject is unlikely to respond to treatment with the Her2-acting agent and/or the patient is likely to have a short time course. In certain preferred embodiments, the Her2-acting agent is trastuzumab.

In another aspect, the invention is drawn to a method for determining whether a subject with a Her-2 positive cancer is unlikely to respond to treatment with at least one chemotherapeutic agent in addition to a Her2-acting agent and/or the patient is likely to have a short time course. In certain embodiments, the method comprises measuring in a biological sample from the subject's cancer an amount of Her-2 and/or Her-2 homodimers, wherein if the level of Her-2 and/or Her-2 homodimers is high and/or very high, then the patient is unlikely to respond to at least one chemotherapeutic agent in addition to a Her-2 acting agent.

In certain embodiments, the biological sample comprises FFPEs. In certain embodiments, the subject's cancer is breast cancer. In certain embodiments, the breast cancer metastatic. In other embodiments, the breast cancer is early stage (i.e., adjuvant) breast cancer. In certain embodiments, the Her-2 acting agent is trastuzumab. In certain embodiments, the chemotherapeutic agent is paclitaxel. In certain embodiments, an amount of total Her-2 is measured. In certain embodiments, an amount of Her-2 homodimers is measured. In certain embodiments, the amount of Her-2 homodimers is measured using an assay capable of measuring and/or quantifying an amount of protein-protein interactions in a sample. In certain embodiment, the assay is the VERATAG® assay. In certain embodiments, likeliness to respond is measured with respect to overall survival rate, time to progression and/or using the RECIST or other response criteria.

In yet another aspect the present disclosure provides a kit for measuring Her-2 and instructions for correlating Her-2 expression to the likelihood that a patient is likely to respond to treatment with a Her2-acting agent. In certain embodiments, the present disclosure also provides kits for predicting a time course of disease and/or a probability of a significant event in the time course of disease in a subject with a cancer based on the predicted sensitivity of a patient to a Her2-acting agent. In certain embodiments the kit comprises reagents to measure additional markers. The other biomarker can be at least one of FOXM1, PRAME, Bc12, STK15, CEGP1, Ki-67, GSTM1, CA9, PR, BBC3, NME1, SURV, GATA3, TFRC, YB-1, DPYD, GSTM3, RPS6KB1, Src, Chk1, ID1, EstR1, p27, CCNB1, XIAP, Chk2, CDC25B, IGF1R, AK055699, P13KC2A, TGFB3, BAGI1, CYP3A4, EpCAM, VEGFC, pS2, hENT1, WISP1, HNF3A, NFKBp65, BRCA2, EGFR, TK1, VDR, Contig51037, pENT1, EPHX1, IF1A, CDH1, HIF1α, IGFBP3; CTSB, Her3 or DIABLO. In certain embodiments, the other biomarker can be VEGF, CD31, KDR, p95, or Her3.

In further aspects of each of the embodiments disclosed herein, the disclosure provides methods of treating a subject with cancer. In one aspect, the methods comprise determining that the subject is afflicted with a cancer that is likely to respond to treatment with a Her-2-acting agent and/or has a long time course according to a method of the invention, and administering an effective amount of a Her-2-acting agent to the subject as a result of said determination. In another aspect, the methods comprise determining that a subject is afflicted with a cancer that is likely to respond to treatment with a Her-2-acting agent according to a method of the invention, then advising a medical professional of the treatment option of administering to the subject an effective amount of a Her-2-acting agent. In another aspect, the methods comprise determining that a subject is afflicted with a cancer that has a short time course and/or that is unlikely to respond to a chemotherapeutic agent in addition to a Her-2 acting agent. In certain embodiments, the Her-2-acting agent is trastuzumab. In certain embodiments, the chemotherapeutic agent is paclitaxel. In certain embodiments, the cancer is breast cancer. In certain embodiments, the breast cancer is metastatic.

### Brief Description of the Drawings

The present invention may be better understood by reference to the following nonlimiting figures.
Figure 1 shows an outline of an FFPE VERATAG® assay in accordance with an embodiment of the present invention.
Figure 2A and Figure 2B show an VERATAG® reaction where diffusing reactive singlet oxygen cleaves the covalent linker between an VERATAG® reporter molecule and an antibody in accordance with alternate embodiments of the present invention.
Figure 3 shows representative electropherograms of the VERATAG® signal generated for four well characterized breast cancer cell lines along with a parallel Her-2 IHC micrograph. The left side of the graph indicates the cell line, the middle shows the corresponding electropherogram and the right side shows the corresponding IHC in accordance with an embodiment of the present invention.
Figure 4 shows the time to progression (TTP) for all HER2 positive patients in accordance with an embodiment of the present invention.
Figure 5 shows the TTP for patients that are HER2 negative or positive determined by FISH detection of Her2 gene amplification in accordance with an embodiment of the present invention.
Figure 6 shows the TTP for patients that are low HER2 expressors that do not respond to treatment with trastuzumab regardless of categorization by FISH measurement of gene amplification in accordance with an embodiment of the present invention.
Figure 7 shows in accordance with an embodiment of the present invention the TTP for patients that are very high HER2 expressors (H2T ≥ 1.84) and/or low HER2 expressors (H2T < 1.14 regardless of FISH) do not respond to treatment with trastuzumab as well as patients having moderately high levels (H2T between 1.14 and 1.84) of HER2 expression.
Figure 8 shows in accordance with an embodiment of the present invention the TTP for patients that have moderately high amounts of HER2 and that are positive as determined by FISH, can be further stratified by HER3 over-expression (H3Thi) or under-expression (H3Tlo) wherein HER3 under-expression is associated with increased responsiveness to treatment with trastuzumab as compared to HER3 over-expression.
Figure 9 shows a study of primary early stage (i.e., adjuvant) Her-2 positive samples in accordance with an embodiment of the present invention, where Panel B shows that CISH positive, very high VERATAG® H2T patients having a cut-off of H2T > 2.21 show little benefit from adding trastuzumab to chemotherapy as compared to samples with Her-2 that is not very high as shown in Panel A.

### Detailed Description of the Invention

As used herein, the terms "embodiment" and "aspect" are used interchangeably.

The term "about," as used herein, unless otherwise indicated, refers to a value that is no more than 10% above or below the value being modified by the term. For example, the term "about 5 µg/kg" means a range of from 4.5 µg/kg to 5.5 µg/kg. As another example, "about 1 hour" means a range of from 48 minutes to 72 minutes.

"Antibody" means an immunoglobulin that specifically binds to, and is thereby defined as complementary with, a particular spatial and polar organization of another molecule. The antibody can be monoclonal, polyclonal, or recombinant and can be prepared by techniques that are well known in the art such as immunization of a host and collection of sera (polyclonal) or by preparing continuous hybrid cell lines and collecting the secreted protein (monoclonal), or by cloning and expressing nucleotide sequences or mutagenized versions thereof coding at least for the amino acid sequences required for specific binding of natural antibodies. Antibodies may include a complete immunoglobulin or fragment thereof, which immunoglobulins include the various classes and isotypes, such as IgA, IgD, IgE, IgG1, IgG2a, IgG2b and IgG3, IgM, etc. Fragments thereof may include Fab, Fv and F(ab')2, Fab', and the like. Antibodies may also be single-chain antibodies or an antigen-binding fragment thereof, chimeric antibodies, humanized antibodies or any other antibody derivative known to one of skill in the art that retains binding activity that is specific for a particular binding site. In addition, aggregates, polymers and conjugates of immunoglobulins or their fragments can be used where appropriate so long as binding affinity for a particular binding site is maintained. Guidance in the production and selection of antibodies and antibody derivatives for use in immunoassays, including such assays employing releasable molecular tag (as described below) can be found in readily available texts and manuals, e.g., Harlow and Lane, 1988, Antibodies: A Laboratory Manual, Cold Spring Harbor Laboratory Press, New York; Howard and Bethell, 2001, Basic Methods in Antibody Production and Characterization, CRC Press; Wild, ed., 1994, The Immunoassay Handbook, Stockton Press, New York.

"Antibody binding composition" means a molecule or a complex of molecules that comprises one or more antibodies, or antigen-binding fragments that bind to a molecule, and derives its binding specificity from such antibody or antibody-binding fragment. Antibody binding compositions include, but are not limited to, (i) antibody pairs in which a first antibody binds specifically to a target molecule and a second antibody binds specifically to a constant region of the first antibody; a biotinylated antibody that binds specifically to a target molecule and a streptavidin protein, which protein is derivatized with moieties such as molecular tags or photosensitizers or the like, via a biotin moiety; (ii) antibodies specific for a target molecule and conjugated to a polymer, such as dextran, which, in turn, is derivatized with moieties such as molecular tags or photosensitizers, either directly by covalent bonds or indirectly via streptavidin-biotin linkages; (iii) antibodies specific for a target molecule and conjugated to a bead, or microbead, or other solid phase support, which, in turn, is derivatized either directly or indirectly with moieties such as molecular tags or photosensitizers, or polymers containing the latter.

"Antigenic determinant," or "epitope" means a site on the surface of a molecule, usually a protein, to which a single antibody molecule binds. Generally, a protein has several or many different antigenic determinants and reacts with antibodies of different specificities. A preferred antigenic determinant is a phosphorylation site of a protein.

"Binding compound" shall refer to an antibody binding composition, an antibody, a peptide, a peptide or non-peptide ligand for a cell surface receptor, a protein, an oligonucleotide, an oligonucleotide analog, such as a peptide nucleic acid, a lectin, or any other molecular entity that is capable of specifically binding to a target protein or molecule or stable complex formation with an analyte of interest, such as a complex of proteins. In one aspect, a binding compound, which can be represented by the formula below, comprises one or more molecular tags attached to a binding moiety.

"Binding moiety" means any molecule to which molecular tags can be directly or indirectly attached that is capable of specifically binding to an analyte. Binding moieties include, but are not limited to, antibodies, antibody binding compositions, peptides, proteins, nucleic acids and organic molecules having a molecular weight of up to about 1000 daltons and containing atoms selected from the group consisting of hydrogen, carbon, oxygen, nitrogen, sulfur and phosphorus. Preferably, binding moieties are antibodies or antibody binding compositions.

"Cancer" and "cancerous" refer to or describe the physiological condition organism, including mammals, that is typically characterized by unregulated cell growth. Examples of cancer include, but are not limited to, carcinoma, lymphoma, blastoma, sarcoma and leukemia. More particular examples of such cancers include squamous cell cancer, lung cancer, *e.g.,* small-cell lung cancer or non-small cell lung cancer; gastrointestinal cancer, pancreatic cancer, glioblastoma, cervical cancer, ovarian cancer, liver cancer, bladder cancer, hepatoma, breast cancer, colon cancer, colorectal cancer, endometrial carcinoma, salivary gland carcinoma, kidney cancer, prostate cancer, vulval cancer, thyroid cancer, hepatic carcinoma and various types of head and neck cancer.

"Chemotherapeutic agent" means a chemical substance, primarily a cytotoxic or cytostatic agent, that is used to treat a condition, particularly cancer. Chemotherapeutic agents shall include such compounds as paclitaxel, as set forth herein.

A "cleavable linkage," as used herein, refers to a chemical linking group that may be cleaved under conditions that do not degrade the structure or affect detection characteristics of a molecular tag connected to a binding moiety with the cleavable linkage.

A "cleavage-inducing moiety," or "cleaving agent," as used herein, is a group that produces an active species that is capable of cleaving a cleavable linkage, preferably by oxidation. Preferably, the active species is a chemical species that exhibits short-lived activity so that its cleavage-inducing effects are only in the proximity of the site of its generation.

A "cleaving probe," as used herein, refers to a reagent that comprises a cleavage-inducing moiety as defined herein and an antibody binding composition, an antibody, a peptide, a peptide or non-peptide ligand for a cell surface receptor, a protein, such as biotin or streptavidin, an oligonucleotide, an oligonucleotide analog, such as a peptide nucleic acid, a lectin or any other molecular entity that is capable of specifically binding to a target protein or molecule or stable complex formation with an analyte of interest, such as a complex of proteins.

"VERATAG®" "VERATAG®" and "VERATAG® assay" are used interchangeably and refer to single and multiplexed and multi-label assays, materials, methods and techniques for performing and utilizing such assays, including but not limited to reagents, analytical. procedures and software related to those assays. Such assays are disclosed in this application as well as in United States Patent 7,105,308.

"FFPE" shall refer to a group of cells or quantity of tissue that are fixed, particularly conventional formalin-fixed paraffin-embedded samples. Such samples are typically, without limitation, used in an assay for receptor complexes in the form of thin sections, *e.g.* 3-10 µm thick, of fixed tissue mounted on a microscope slide or equivalent surface. Such samples also typically undergo a conventional re-hydration procedure, and optionally, an antigen retrieval procedure as a part of, or preliminary to, assay measurements.

"Hazard ratio", as used herein, refers to a statistical method used to generate an estimate for relative risk. "Hazard ratio" is the ratio between the predicted hazard of one group versus another group. For example, patient populations treated with versus without a Her-2 acting agent can be assessed for whether or not the Her-2 acting agent is effective in increasing the time to distant recurrence of disease. The hazards ratio can then be compared to an independent measure, such as the ratio of Her-2 homodimer to total Her-2. At Her-2 homodimer to total Her-2 ratios at which the hazards ratio is less than one, treating with a Her-2 acting agent has a greater chance of efficacy. At Her-2 homodimer to total Her-2 ratios at which the hazards ratio is indistinguishable from one, treating with a Her-2 acting agent has a lower chance of efficacy.

"Her-2", "ErbB2", "c-Erb-B2", "HER2", "Her2" and *"neu"* are used interchangeably herein and refer to native Her-2, and allelic variants thereof, as described, for example, in Semba et al., 1985, P.N.A.S. USA 82:6497-650 and Yamamoto et al., 1986, Nature 319:230-234 and Genebank accession number X03363. Unless indicated otherwise, the terms "Her-2", "ErbB2", "c-Erb-B2", "HER2" and "Her2" when used herein refer to the human protein. The gene encoding Her2 is referred to herein as "erbB2." As used herein, H2T shall refer to total Her-2 expression as shown, for example without limitation, by VERATAG®.

"Her-2-acting agent," as used herein, refers to a compound that can inhibit a biological activity of Her-2 or a Her-2 expressing cell or a Her-2 positive cancer cell. Such biological activities include, but are not limited to, dimerization, autophosphorylation, phosphorylation of another receptor, signal transduction and the like. Biological activities can include, without limitation, cell survival and cell proliferation, and inhibition of such activities by a Her-2 acting agent could be direct or indirect cell killing (eg, ADCC), disruption of protein complexes or complex formation, modulation of protein trafficking or enzyme inhibition. Biological activities can also include patient response as set forth in this application. Exemplary Her-2-acting agents include, but are not limited to, the large molecules 4D5 and trastuzumab and small molecules such as AEE-788 and lapatinib. "Her-2 homodimer" in reference to cell surface Her-2 membrane receptors means a complex of two or more membrane-bound Her-2 proteins. Dimers usually consist of two receptors in contact with one another. Dimers may be created in a cell surface membrane by passive processes, such as Van der Waal interactions, and the like, or dimers may be created by active processes, such as by ligand-induced dimerization, covalent linkages, interaction with intracellular components or the like. *See, e.g.,* Schlessinger, 2000, Cell 103:211-225. As used herein, the term "dimer" is understood to refer to "cell surface membrane receptor dimer," unless understood otherwise from the context. As used herein, H22D shall refer to quantified dimer as shown, for example without limitation, by VERATAG®.

"Her-2 homodimer to total Her-2 ratio" refers to a measure that describes the amount of Her-2 homodimers divided by the total amount of Her-2 in a sample from a subject's tissue according to any single quantitative method available to one skilled in the art.

A "Her-2 positive" cancer, cancer cell, subject or patient, as used herein, refers to a cancer, cell subject or patient exhibiting a score of at least 2 when using a HercepTest^{®} (DakoCytomation California Inc., Carpenteria, CA) or a cancer, cancer cell, subject or patient that has been identified as such by FISH. In certain embodiments, the Her-2 positive cell exhibits a score of at least 2+ or 3+ using HercepTest^{®}.

"High" refers to a measure that is greater than normal, greater than a standard such as a predetermined measure or a subgroup measure or that is relatively greater than another subgroup measure. For example, high Her-2 refers to a measure of Her-2 that is greater than a normal Her-2 measure. A normal Her-2 measure may be determined according to any method available to one skilled in the art. High Her-2 may also refer to a measure that is equal to or greater than a predetermined measure, such as a predetermined cutoff. High Her-2 may also refer to a measure of Her-2 wherein a high Her-2 subgroup has relatively greater levels of Her-2 than another subgroup. For example, without limitation, according to the present specification, two distinct patient subgroups can be created by dividing samples around a mathematically determined point, such as, without limitation, a median, thus creating a subgroup whose measure is high (ie, higher than the median) and another subgroup whose measure is low. Her-2 can be measured by any method known to one skilled in the art such as, for example, without limitation, using VERATAG® or using any standard immunohistochemical (IHC) method such as HercepTest^{®}. As another example, high Her-2 homodimers refers to a measure of Her-2 homodimers that is greater than a normal measure of Her-2 homodimers in a particular set of samples or patients that are Her-2 positive. A normal Her-2 homodimer measure may be determined according to any method available to one skilled in the art. High Her-2 homodimers may also refer to a measure that is greater than a predetermined measure, such as a predetermined cutoff. High Her-2 homodimers may also refer to a measure of Her-2 homodimers wherein a high Her-2 homodimer subgroup has a relatively higher level of Her-2 homodimers than another subgroup. Her-2 homodimers can be measured by any method known in the art such as Fluorescence resonance energy transfer (FRET), Biolumenescent resonance energy transfer (BRET), proximity ligation assay (PLA), dimer-specific antibodies or VERATAG® or any other method that is well known to one skilled in the art. As another example, high Her-2 homodimer to total Her-2 ratio may refer to the one or more subgroups of Her-2 homodimer to total Her-2 ratios that have measures greater than either intermediate or low ratio subgroups. High Her-2 homodimer to total Her-2 ratios may be determined according to any individual quanitative method available to one skilled in the art. In some cases, a "high" expression level may comprise a range of expression that is very high and a range of expression that is "moderately high" where moderately high is a level of expression that is greater than normal, but less than "very high". Example ranges for high (including very high and moderately high) Her-2 expression are provided herein.

"Moderately high" "medium" or "intermediate", as used herein, refers to a measure that is greater than "low" and less than very "high". For example, "intermediate" may be used to describe one or more of the at least 3 subgroups that fall in the middle range of measures of Her-2 homodimer to total Her-2 ratios.

"Likely to," as used herein, refers to an increased probability that an item, object, thing or person will occur. Thus, in one example, a subject that is likely to respond to treatment with trastuzumab has an increased probability of responding to treatment with trastuzumab relative to a reference subject or group of subjects.

"Long," as used herein, refers to a time measure that is greater than normal, greater than a standard such as a predetermined measure or a subgroup measure that is relatively longer than another subgroup measure. For example, with respect to a patient's longevity, a long time progression refers to time progression that is longer than a normal time progression. Whether a time progression is long or not may be determined according to any method available to one skilled in the art. Long could include, for example, no progression. In one embodiment, "long" refers to a time that is greater than the median time course required for a significant event to occur in a disease.

"Low" is a term that refers to a measure that is less than normal, less than a standard such as a predetermined measure or a subgroup measure that is relatively less than another subgroup measure. For example, low Her-2 means a measure of Her-2 that is less than a normal Her-2 measure in a particular set of samples of patients that is Her-2 positive. A normal Her-2 measure may be determined according to any method available to one skilled in the art. Low Her-2 may also mean a method that is less than a predetermined measure, such as a predetermined cutoff. Low Her-2 may also mean a measure wherein a low Her-2 subgroup is relatively lower than another subgroup. For example, without limitation, according to the present specification, two distinct patient subgroups can be created by dividing samples around a mathematically determined point, such as, without limitation, a median, thus creating a group whose measure is low (ie, less than the median) with respect to another group whose measure is high. Her-2 can be measured by any method known to one skilled in the art such as, for example, without limitation, using the VERATAG® method or using any standard immunohistochemical (IHC) method such as HercepTest^{®}. As another example, low Her-2 homodimers means a measure of Her-2 homodimers that is less than a normal measure of Her-2 homodimers in a particular set of samples or patients that is Her-2 positive. Low Her-2 homodimers may also mean a measure that is less than a predetermined measure, such as a predetermined cutoff. Low Her-2 homodimers may also mean a measure wherein a low Her-2 homodimer subgroup is relatively less than another subgroup. Her-2 homodimers can be measured by any method known in the art such as Fluorescence resonance energy transfer (FRET), Biolumenescent resonance energy transfer (BRET), proximity ligation assay (PLA), dimer-specific antibodies or VERATAG® or any other method that is well known to one skilled in the art. As another example, low Her-2 homodimer to total Her-2 ratio may refer to the one or more subgroups of Her-2 homodimer to total Her-2 ratios that have measures less than either intermediate or high ratio subgroups. Low Her-2 homodimer to total Her-2 ratios may be determined according to any individual quanitative method available to one skilled in the art. Example ranges for low values of Her-2 expression are provided herein.

A "molecular tag," as used herein, refers to a molecule that can be distinguished from other molecules based on one or more physical, chemical or optical differences among the molecules being separated, including but not limited to, electrophoretic mobility, molecular weight, shape, solubility, pKa, hydrophobicity, charge, charge/mass ratio, polarity or the like. In one aspect, molecular tags in a plurality or set differ in electrophoretic mobility and optical detection characteristics and can be separated by electrophoresis. In another aspect, molecular tags in a plurality or set may differ in molecular weight, shape, solubility, pKa, hydrophobicity, charge, polarity and can be separated by normal phase or reverse phase HPLC, ion exchange HPLC, capillary electrochromatography, mass spectroscopy, gas phase chromatography or like technique.

"Optimal cutoff 'as used herein, refers to the value of a predetermined measure on subjects exhibiting certain attributes that allow the best discrimination between two categories of an attribute. For example, finding a value for an optimal cutoff that allows one to best discriminate between two categories, high H2T expression and low H2T expression, for determining OS. Optimal cutoffs are used to separate the subjects with values lower than or higher than the optimal cutoff to optimize the prediction model, for example, without limitation, to maximize the specificity of the model, maximize the sensitivity of the model, maximize the difference in outcome, or minimize the p-value from hazard ratio or a difference in response.

"Overall survival" or "OS" refers to a time as measured from the start of treatment to death or censor. Censoring may come from a study end or change in treatment. Overall survival can refer to a probability as, for example, a probability when represented in a Kaplan-Meier plot of being alive at a particular time, that time being the time between the start of the treatment to death or censor.

"Photosensitizer" shall mean a light-adsorbing molecule that when activated by light converts molecular oxygen into singlet oxygen.

"RECIST" shall mean an acronym that stands for "Response Evaluation Criteria in Solid Tumours" and is a set of published rules that define when cancer patients improve ("respond"), stay the same ("stable") or worsen ("progression") during treatments. Response as defined by RECIST criteria have been published, for example, at Journal of the National Cancer Institute, Vol. 92, No. 3, February 2, 2000 and RECIST criteria may include other similar published definitions and rule sets. One skilled in the art would understand definitions that go with RECIST criteria, as used herein, such as "PR," "CR," "SD" and "PD."

"Relative fluorescence units" or "RFUs" are used interchangeably and shall refer to the time integral of a particular capillary electrophoresis peak using arbitrary fluorescence units in comparison to a standard. With respect to VERATAG®, the RFU is proportional to the concentration of VERATAG® injected into capillary electrophoresis with some expected variability introduced by, for example, injection and capillary differences.

"Relative peak area" or "RPA" are used interchangeably and shall refer to the ratio between an RFU of a particular VERATAG® and an RFU of a known internal fluorescence standard of known and constant concentration.

"Responsiveness," to "respond" to treatment, and other forms of this verb, as used herein, refer to the reaction of a subject to treatment with a Her-2-acting agent. As an example, a subject responds to treatment with a Her2-acting agent if growth of a tumor in the subject is retarded about 10%, 20%, 30%, 40%, 50%, 60%, 70%, 80%, 90% or more. In another example, a subject responds to treatment with a Her-2-acting agent if a tumor in the subject shrinks by about 5%, 10%, 20%, 30%, 40%, 50% or more as determined by any appropriate measure, e.g., by mass or volume. In another example, a subject responds to treatment with a Her2-acting agent if the subject experiences a life expectancy extended by about 5%, 10%, 20%, 30%, 40%, 50% or more beyond the life expectancy predicted if no treatment is administered. In another example, a subject responds to treatment with a Her-2-acting agent if the subject has an increased disease-free survival, overall survival or increased time to progression. Several methods may be used to determine if a patient responds to a treatment including the RECIST criteria, as set forth above.

"Sample" or "tissue sample" or "patient sample" or "patient cell or tissue sample" or "specimen" each refer to a collection of similar cells obtained from a tissue of a subject or patient. The source of the tissue sample may be solid tissue as from a fresh, frozen and/or preserved organ or tissue sample or biopsy or aspirate; blood or any blood constituents; bodily fluids such as cerebral spinal fluid, amniotic fluid, peritoneal fluid or interstitial fluid; or cells from any time in gestation or development of the subject. The tissue sample may contain compounds that are not naturally intermixed with the tissue in nature such as preservatives, anticoagulants, buffers, fixatives, nutrients, antibiotics or the like. Cells may be fixed in a conventional manner, such as in an FFPE manner.

"Short," as used herein, refers to a time measure that is shorter than normal, shorter than a standard such as a predetermined measure or a subgroup measure that is relatively shorter than another subgroup measure. For example, with respect to a patient's longevity, a short time progression refers to time progression that is shorter than a normal time progression. Whether a time progression is short or not may be determined according to any method available to one skilled in the art. In one embodiment, "short" refers to a time that is less than the median time course required for a significant event to occur in a disease.

As used herein, "significant event" shall refer to an event in a patient's disease that is important as determined by one skilled in the art. Examples of significant events include, for example, without limitation, primary diagnosis, death, recurrence, the determination that a patient's disease is metastatic, relapse of a patient's disease or the progression of a patient's disease from any one of the above noted stages to another. A significant event may be any important event used to assess OS, TTP and/or using the RECIST or other response criteria, as determined by one skilled in the art.

As used herein, the terms "subject" and "patient" are used interchangeably. As used herein, the terms "subject" and "subjects" refer to an animal, preferably a mammal including a non-primate (e.g., a cow, pig, horse, donkey, goat, camel, cat, dog, guinea pig, rat, mouse, sheep) and a primate *(e.g.,* a monkey, such as a cynomolgous monkey, gorilla, chimpanzee and a human).

As used herein, "time course" shall refer to the amount of time between an initial event and a subsequent event. For example, with respect to a patient's cancer, time course may relate to a patient's disease and may be measured by gauging significant events in the course of the disease, wherein the first event may be diagnosis and the subsequent event may be metastasis, for example.

"Time to progression" or "TTP" refers to a time as measured from the start of the treatment to progression or a cancer or censor. Censoring may come from a study end or from a change in treatment. Time to progression can also be represented as a probability as, for example, in a Kaplein-Meier plot where time to progression may represent the probability of being progression free over a particular time, that time being the time between the start of the treatment to progression or censor.

"Treat," "treatment," and other forms of this word refer to the administration of a Her-2-acting agent to impede growth of a cancer, to cause a cancer to shrink by weight or volume, to extend the expected survival time of the subject and or time to progression of the tumor or the like.

"Unlikely to" refers to a decreased probability that an event, item, object, thing or person will occur with respect to a reference. Thus, a subject that is unlikely to respond to treatment with paclitaxel in addition to trastuzumab has a decreased probability of responding to treatment with paclitaxel and trastuzumab relative to a reference subject or group of subjects.

Thus, embodiments of the invention provide methods for determining whether a subject with a cancer is likely to respond to treatment with a Her-2-acting agent and/or for predicting a time course of disease and/or a probability of a significant event in the time course of disease in a subject with a cancer. In certain embodiments, the method comprises detecting a biomarker or combination of biomarkers associated with responsiveness to treatment with a Her-2-acting agent as described herein, and determining whether the subject is likely to respond to treatment with the Her2-acting agent. In certain embodiments, the methods comprise detecting a biomarker or combination of biomarkers and predicting a time course associated with progression of disease or a probability of a significant event in the time course of disease in a subject with cancer.

In one aspect, the invention is drawn to a method for determining whether a subject with a cancer is likely to respond to treatment with a Her-2-acting agent. In another aspect, the invention is drawn to a method for predicting a time course of disease. In another aspect, the method is drawn to a method for predicting a probability of a significant event in the time course of the disease.

In certain embodiments, a time course is measured by determining the time between significant events in the course of a patient's disease, wherein the measurement is predictive of whether a patient has a long time course. For example, in a preferred embodiment, the significant event is the progression from primary diagnosis to death. In a preferred embodiment, the significant event is the progression from primary diagnosis to metastatic disease. In a preferred embodiment, the significant event is the progression from primary diagnosis to relapse. In a preferred embodiment, the significant event is the progression from metastatic disease to death. In a preferred embodiment, the significant event is the progression from metastatic disease to relapse. In a preferred embodiment, the significant event is the progression from relapse to death. In certain embodiments, the time course is measured with respect to overall survival rate, time to progression and/or using the RECIST or other response criteria.

In certain embodiments, the method comprises detecting in a biological sample from the subject's cancer the amount of Her-2 and/or Her-2 homodimers wherein if the amount of Her-2 and/or Her-2 homodimers is high, then the patient is likely to respond to the Her-2 acting agent and/or the patient has a long time course.

Thus, in certain embodiments, the disclosure comprises methods to correlate the relative levels of the amount of at least one of Her-2 or Her-2 homodimers in a biological sample from a subject with a prognosis for the likelihood that the subject will respond to treatment with a Her-2 acting agent comprising: (a) detecting in a biological sample from the subject's cancer the amount of at least one of Her-2 or Her-2 homodimers; and (b) correlating the amount of Her-2 or Her-2 homodimers to a prognosis for the likelihood that the subject will respond to treatment with a Her-2 acting agent.

In certain embodidments, if the amount of the at least one of the Her-2 or Her-2 homodimers is equal to or above a first threshold level, the subject's prognosis is to be likely to respond to the Her-2 acting agent. Additionally and/or alternatively, if the amount of the at least one of the Her-2 or Her-2 homodimers is equal to or above a second threshold level higher than the first threshold level, the subject's prognosis is to be unlikely to respond to the Her-2 acting agent

Also, in certain embodiments, a predetermined measure is created by dividing a plurality of subject samples into at least three subgroups, wherein the first subgroup comprises samples having the Her-2 or Her-2 homodimers at a low level, wherein the low level comprises having an amount of the at least one of the Her-2 or Her-2 homodimers is equal to or below a first threshold level, and the samples having an amount of the at least one of the Her-2 or Her-2 homodimers equal to or above the first threshold comprise samples having a high level of the Her-2 or Her-2 homodimers, and wherein the samples having a high level of the Her-2 or Her-2 homodimers is then divided into two subgroups, a very high subgroup comprising samples having an amount of the at least one of the Her-2 or Her-2 homodimers equal to or above a second threshold level higher than the first threshold level, and a moderately high subgroup comprising samples having an amount of the at least one of the Her-2 or Her-2 homodimers greater than or equal to the first threshold level and less than or equal to the second threshold level.

As discussed herein, in certain embodiments, the amount of Her-2 homodimers is measured using an assay capable of measuring and/or quantifying an amount of protein-protein interactions in a sample.

In certain embodiments of each of the methods of the present invention, high Her-2 expression is a log10H2T ≥ about 1.14-1.125. In certain embodiments of each of the methods disclosed herein, the high Her-2 expression comprises expression that is very high and/or moderately high. In certain embodiments of each of the methods disclosed herein, the very high Her-2 expression is a log10H2T ≥ about 1.84 -2.21. In certain embodiments of each of the methods disclosed herein, the moderately high expression is between 1.14 - 1.25 and 1.84-2.21. Or, other ranges (i.e., up to about 25% more or less as described herein) may be used depending upon the patient cohort and/or the significant event being monitored.

In certain embodiments, the cancer is breast cancer. In some embodiments, the breast cancer is metastatic. In some embodiments, the breast cancer is early stage (i.e., adjuvant) breast cancer. Or, any cancer that may be sensistive to a Her-2 acting agent may be monitored. The Her-2 acting agent may be any Her-2 acting agent. In certain embodiments, the Her-2 acting agent is one of the agents described herein. For example, in certain embodiments, the Her-2 acting agent is trastuzumab.

In certain embodiments of each of the methods of the invention, the moderately high Her-2 subgroup is further subdivided into subgroups that express at least one other biomarker. For example, in some embodiments, a predetermined measure is generated by dividing the Her-2 medium and/or Her-2 high samples into at least two subgroups, based on the level of the at least one other biomarker

The at least one other biomarker may, in alternate embodiments, comprise at least one of FOXM1, PRAME, Bc12, STK15, CEGP1, Ki-67, GSTM1, CA9, PR, BBC3, NME1, SURV, GATA3, TFRC, YB-1, DPYD, GSTM3, RPS6KB1, Src, Chk1, ID1, EstR1, p27, CCNB1, XIAP, Chk2, CDC25B, IGF1R, AK055699, P13KC2A, TGFB3, BAGI1, CYP3A4, EpCAM, VEGFC, pS2, hENT1, WISP1, HNF3A, NFKBp65, BRCA2, EGFR, TK1, VDR, Contig51037, pENT1, EPHX1, IF1A, CDH1, HIF1α, IGFBP3; CTSB, Her3, DIABLO, VEGF, CD31, KDR, or p95.

For example, in certain embodiments, the moderately high subgroup is further divided based upon Her-3 expression wherein a high level comprises having Her-3 equal to or above a first threshold level and a low level comprises having Her-3 below the first threshold level, and wherein a subject with moderately high levels of the at least one Her-2 and/or Her-2 dimers and low levels of Her-3 is likely to respond to the Her-2 acting agent and/or wherein a subject with moderately high levels of the at least one Her-2 and/or Her-2 dimers and high levels of Her-3 is unlikely or less likely to respond to the Her-2 acting agent.. Where Her-3 is measured, the Her-3 expression comprises Her-3, Her-3 homodimers, or Her-3 heterodimers (e.g., Her-2/Her-3).

In certain embodiments, the assay is performed with an VERATAG® assay. In certain embodiments, likeliness to respond is measured with respect to overall survival rate, time to progression and/or using the RECIST or other response criteria.

In certain embodiments, the method comprises detecting in a biological sample from the subject's cancer the amount of Her-2 and/or Her-2 homodimers wherein if the amount of Her-2 and/or Her-2 homodimers is moderately high (e.g., medium), then the patient group may be further sub-divided into high Her-3 expressors and low Her-3 expressors. In this embodiment, the patient with moderately high Her-2 and/or Her-2 homodimers and low Her-3 is likely to respond to the Her-2 acting agent and/or the patient has a long time course. In alternate embodiments, the Her-3 expressors can be expression of Her-3, Her-3 homodimers, or Her-2/Her3 heterodimers.

In certain embodiments of measuring both Her2 and Her3, the cancer is breast cancer. In some embodiments, the breast cancer is metastatic. In other embodiments, the breast cancer is early stage (i.e., adjuvant) breast cancer. Or, other Her-2 sensitive cancers may be monitored. The Her-2 acting agent may be any Her-2 acting agent. In certain embodiments, the Her-2 acting agent is one of the agents described herein. For example, iIn certain embodiments, the Her-2 acting agent is trastuzumab. In certain embodiments, the assay is performed with an VERATAG® assay. In certain embodiments, likeliness to respond is measured with respect to overall survival rate, time to progression and/or using the RECIST or other response criteria.

In certain embodiments, a predetermined measure is created by dividing patient samples into at least two patient subgroups. In certain embodiments, the number of subgroups is three so that the patient sample is divided into a subgroup of patients whose Her-2 and/or Her-2 homodimers is very high, a subgroup whose Her-2 and/or Her-2 homodimers is low, and a subgroup whose Her-2 and/or Her-2 homodimers is moderately high (i.e., medium). Each of the sub-groups can then be further subdivided into a subgroup whose Her-3 is high or medium.

The level of Her-2 homodimers may be tightly correlated to the total leverls of Her-2. Thus, in certain embodiments of each of the methods of the present invention, the amount of Her-2 and/or Her-2 homodimers in the subject are compared to at least one of the very high subgroup, the moderately high subgroup, or the low subgroup. If the amount of Her-2 and/or Her-2 homodimers in the patient are moderately high, and the Her-3 expressors are low, then the patient is likely to respond to a Her-2 acting agent and/or the patient is likely to have a long time course. If the amount of Her-2 and/or Her-2 homodimers in the patient are moderately high, and the Her-3 expressors are high, then the patient is unlikely to respond to a Her-2 acting agent and/or the patient is likely to have a short time course. In certain embodiments, the number of subgroups is greater than two, including, without limitation, three subgroups, four subgroups, five subgroups and six subgroups. In certain embodiments, likeliness to respond is measured with respect to overall survival rate, time to progression and/or using the RECIST criteria. In certain preferred embodiments, the Her-2 acting agent is trastuzumab.

In certain embodiments, the predetermined measure is an optimal cutoff. Such optimal cutoffs are disclosed herein, and certain embodiments of the invention are meant to include amounts that are approximate to the amounts mentioned and disclosed herein. In certain embodiments, the amount of Her-2 and/or Her-2 homodimers in the subject are compared to the optimal cutoff, if the amount of Her-2 and/or Her-2 homodimers in the patient are moderately high (e.g., between about 1.14-1.25 and 1.84-2.21 H2T), then the patient is likely to respond to a Her-2 acting agent and/or the patient's time course is likely to be long. In another embodiment, if the amount of Her-2 is high, then the patient is likely to respond to a Her-2 acting agent and/or the time course is likely to be long. In another embodiment, if the amount of Her-2 is high, and the amount of Her-2 homodimers and/or the ratio of Her-2 homodimers to Her-2 are low, then the patient is likely to respond to a Her-2 acting agent and/or the time course is likely to be long. In another embodiment, if the amount of Her-2 is high and the amount of Her-2 dimers is high, then the patient is likely to respond to a Her-2 acting agent and/or the time course is likely to be long.

In another aspect, the disclosure is drawn to a method for determining whether a subject with a Her-2 positive cancer is likely to respond to treatment with a Her-2-acting agent and/or the time course of disease is long. In another aspect, the invention is drawn to a method for predicting a time course of disease in a subject with a Her-2 positive cancer. In another aspect, the invention is drawn to a method for predicting the probability of a significant event in a subject with a Her-2 positive cancer.

For example, the disclosure comprises methods for predicting whether a subject with a cancer and being treated with a Her-2 acting agent is likely to have a significant event comprising the steps of: (a) detecting in a biological sample from the subject's cancer the amount of at least one of Her-2 or Her-2 homodimers; and (b) correlating the amount of Her-2 or Her-2 homodimers to the likelihood that the subject will have a significant event.

In an embodiment, the significant event is a reduced time between diagnosis with the cancer and at least one of primary diagnosis, progression of the cancer from one stage to a more advanced stage, progression to metastatic disease, relapse, surgery or death. Also in certain embodiments, the method may further comprise predicting a time course during which the significant event can occur.

In an embodiment, if the amount of the at least one of the Her-2 or Her-2 homodimers is equal to or below a first threshold level, the significant event is that the subject is less likely to respond to the Her-2 acting agent. Additionally and/or alternatively, if the amount of the at least one of the Her-2 or Her-2 homodimers is equal to or above a second threshold level higher than the first threshold level, the subject's prognosis is to be unlikely to respond to the Her-2 acting agent.

In a preferred embodiment, a time course is measured by determining the time between significant events in the course of a patient's disease, wherein the measurement is predictive of whether a patient has a long time course. In one embodiment, the significant event is the progression from primary diagnosis to death. In another embodiment, the significant event is the progression from primary diagnosis to metastatic disease. In yet another embodiment, the significant event is the progression from primary diagnosis to relapse. In another embodiment, the significant event is the progression from metastatic disease to death. In another embodiment, the significant event is the progression from metastatic disease to relapse. In another embodiment, the significant event is the progression from relapse to death. In certain embodiments, the time course is measured with respect to overall survival rate, time to progression and/or using the RECIST or other response criteria.

In certain embodiments, the method comprises measuring in a biological sample from the subject's cancer an amount of Her-2 and/or Her-2 homodimers, wherein if the amount of Her-2 and/or Her-2 homodimers is high and/or moderately high, then the patient is likely to respond to the Her-2 acting agent and/or the patient has a long time course. In certain embodiments, the biological sample comprises FFPEs. In certain embodiments, the subject's cancer is breast cancer. In certain embodiments, the breast cancer is metastatic. In other embodimetns, the cancer is early stage (i.e., adjuvant) breast cancer. Or, other cancers sensitive to Her-2 aciting agents may be monitored. As noted herein, the Her-2 acting agent may be one of the agents known. In certain embodiments, the Her-2-acting agent is trastuzumab. Or other Her-2 acting agents may be used.

In certain embodiments, an amount of Her-2 is measured. In certain embodiments, an amount of Her-2 homodimers is measured. In certain embodiments, the amount of Her-2 homodimers is measured using an assay capable of measuring and/or quantifying an amount of protein-protein interactions in a sample. In certain embodiment, the assay is the VERATAG® assay. In certain embodiments, likeliness to respond is measured with respect to overall survival rate, time to progression and/or using the RECIST criteria.

In certain embodiments, the method comprises measuring in a biological sample from the subject's cancer an amount of Her-3 and/or Her-3 homodimers as well as the amount of Her-2 and/or Her-2 homodimers, wherein if the amount of Her-3 and/or Her-3 homodimers is high, then the patient is likely to respond to the Her-2 acting agent and/or the patient has a long time course. In certain embodiments, the biological sample comprises FFPEs. In certain embodiments, the subject's cancer is breast cancer. In certain embodiments, the breast cancer is metastatic. Or the breast cancer may be an early stage (i.e., adjuvant) breast cancer. Or, other cancers sensitive to Her-2 aciting agents may be monitored. As noted herein, the Her-2 acting agent may be one of the agents known and/or described herein. In certain embodiments, the Her-2-acting agent is trastuzumab.

In certain embodiments, an amount of Her-3 homodimers is measured. In certain embodiments, the amount of Her-3 homodimers is measured using an assay capable of measuring and/or quantifying an amount of protein-protein interactions in a sample. In certain embodiment, the assay is the VERATAG® assay. In certain embodiments, likeliness to respond is measured with respect to overall survival rate, time to progression and/or using the RECIST criteria.

In certain embodiments, the method comprises measuring in a biological sample from the subject's cancer an amount of Her-2 and/or Her-2 homodimers, wherein if the amount of Her-2 and/or Her-2 homodimers is moderately high (i.e., medium), then the biological sample is further analyzed for the amount of Her-3 expressors, wherein the Her-3 expressors can be expression of Her-3, Her-3 homodimers, or Her-2/Her3 heterodimers. If the amount of Her-2 and/or Her-2 homodimers in the patient is moderately high and the amount of Her-3 expressors is high, then the patient is likely to respond to the Her-2 acting agent and/or the patient has a long time course. Conversely, if the amount of Her-2 and/or Her-2 homodimers in the patient is moderately high and the amount of Her-3 expressors is low, then the patient is unlikely to respond to the Her-2 acting agent and/or the patient has a short time course. In certain embodiments, the biological sample comprises FFPEs. In certain embodiments, the subject's cancer is breast cancer. In certain embodiments, the breast cancer is metastatic. Or, the breast cancer may be early stage (i.e., adjuvant) breast cancer. Or, any other cancer responsive to Her-2 acting agents may be monitored. As noted herein, the Her-2 acting agent may be one of the agents known and/or described herein. In certain embodiments, the Her-2-acting agent is trastuzumab. In certain embodiment, the assay is the VERATAG® assay. In certain embodiments, likeliness to respond is measured with respect to overall survival rate, time to progression and/or using the RECIST criteria.

In certain embodiments, a predetermined measure is created by dividing patient samples into at least two patient subgroups. In certain embodiments, the number of subgroups is three so that the patient sample is divided into a subgroup of patients whose Her-2 and/or Her-2 homodimers is high, and a subgroup whose Her-2 and/or Her-2 homodimers is low. In certain embodiments, the high Her-2 subgroup is divided into a a subgroup whose Her-2 and/or Her-2 homodimers is very high, and a subgroup whose Her-2 and/or Her-2 homodimers is moderately hight (i.e., medium). In an embodiment; the amount of Her-2 and/or Her-2 homodimers in the subject are compared to either the very high subgroup, the moderately high subgroup, or the low subgroup. If the amount of Her-2 and/or Her-2 homodimers in the patient are moderately high, then the patient is likely to respond to a Her-2 acting agent and/or the patient is likely to have a long time course. If the amount of Her-2 and/or Her-2 homodimers in the patient are very high or low, then the patient is unlikely to respond to a Her-2 acting agent and/or the patient is likely to have a short time course.

In another embodiment, if the amount of Her-2 and/or Her-2 homodimers in the patient is moderately high and the amount of Her-3 expressors is high, then the patient is unlikely to respond to the Her-2 acting agent and/or the patient has a short time course. In another embodiment, if the amount of Her-2 and/or Her-2 homodimers in the patient is moderately high and the amount of Her-3 expressors is low, then the patient is likely to respond to the Her-2 acting agent and/or the patient has a long time course. In certain embodiments, the number of subgroups is greater than three, including, without limitation, four subgroups, five subgroups and six subgroups. In certain embodiments, likeliness to respond or time course is measured with respect to overall survival rate, time to progression and/or using the RECIST criteria. In certain preferred embodiments, the Her-2 acting agent is trastuzumab.

In certain embodiments, the predetermined measure is an optimal cutoff. Such optimal cutoffs are disclosed herein, and certain embodiments of the invention are meant to include amounts that are approximate to the amounts mentioned and disclosed herein. In certain embodiments, the amount of Her-2 and/or Her-2 homodimers in the subject are compared to the optimal cutoff; if the amount of Her-2 and/or Her-2 homodimers in the patient are high or moderately high, then the patient is likely to respond to a Her-2 acting agent and/or the patient's time course is likely to be long. In another embodiment, if the amount of Her-2 is high, and the amount of Her-2 homodimers and/or the ratio of Her-2 homodimers to Her-2 is low, then the patient is likely to respond to a Her-2 acting agent and/or the time course is likely to be long. In another embodiment, if the amount of Her-2 is high and the amount of Her-2 homodimers and/or the ratios of Her-2 homodimers to Her-2 is high, then the patient is likely to respond to a Her-2 acting agent and/or the time course is likely to be long.

In another aspect, the invention provides a method for determining whether a subject with Her2-positive cancer is unlikely to respond to treatment with a Her2-acting agent and/or the patient is likely to have a short time course. In certain embodiments, the method comprises detecting in a biological sample from the subject's cancer the amount of Her-2, wherein if the amount of Her-2 is low, the subject is unlikely to respond to treatment with the Her2-acting agent and/or the patient is likely to have a short time course. In certain preferred embodiments, the Her2-acting agent is trastuzumab.

Any method known to one of skill in the art to be useful for determining an amount of Her-2 expression and/or Her-2 homodimers, and/or Her-3 expression and/or Her-3 homodimers, and/or Her-2/Her-3 heterodimers can be used in accordance with the present invention. For example, any quantitative assay that determines the amount of such expression or dimers can be used to determine how much signal is generated by a cell or cancer, then the signal compared to the signal generated in the VERATAG^{®} assay to determine a correspondence between the two assays. Such methods may include, but not necessarily be limited to, FRET, BRET, Biomolecular Fluoresence Complementation and Proximity Ligation Assay.

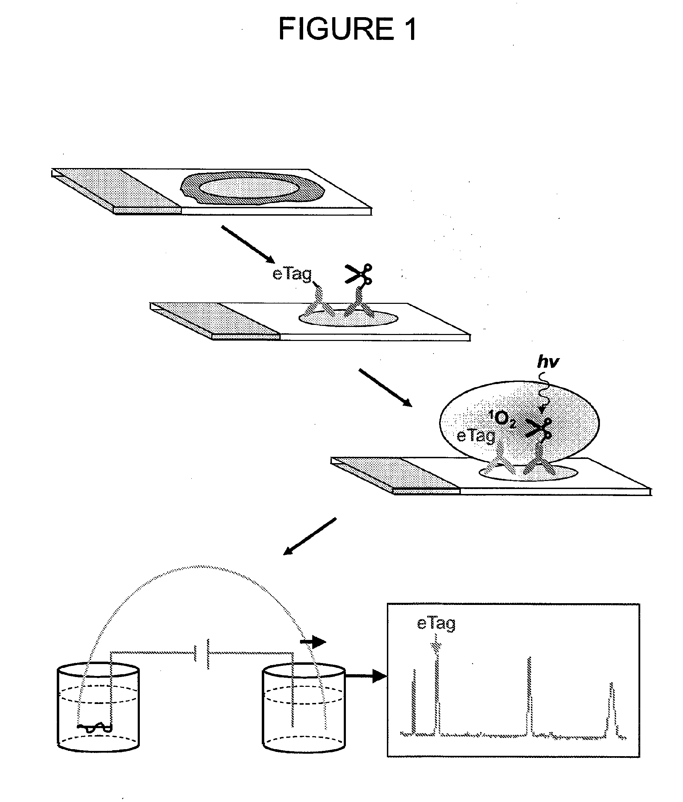

In certain embodiments, the amounts are determined by contacting a biological sample from a subject with cancer with a binding compound having a molecular tag attached thereto by a cleavable linkage and a cleaving probe having a cleavage inducing-moiety and detecting whether and what molecular tag is released. Figure 1 provides an outline of such an FFPE VERATAG® assay where tissue sections are fixed (top or first panel) and then allowed to bind to a first antibody having a cleavage-inducing agent (depicted as a cutting tool) and a second antibody linked to a detectable moiety (ETAG®) (second panel), photo-induction of the cleavage-inducing agent by light (hv) (third panel), electrophoretic separation of the e-Tag(s) (fourth panel) and a readout of the data (bottom or fifth panel).

In certain embodiments, the binding compound and the cleaving probe each specifically binds Her-2 or Her-3. In certain embodiments, the cleaving probe and the binding probe do not both bind the same epitope. In certain embodiments, if the binding compound is within an effective proximity of the cleavage-inducing moiety of the cleaving probe, the cleavage-inducing moiety cleaves the cleavable linker so that the molecular tag is released. In certain embodiments, the molecular tag released if Her-2 homodimers, Her-3 homodimers, or Her-2/Her-3 heterodimers are present is distinguishable from the molecular tag released if Her-2 monomers and/or Her-3 monomers are present. Examples of detection of Her-2 by an assay for detection of total Her-2 and/or Her-2 homodimers is provided in commonly owned U.S. Patent Application Publication No. 2009/0191559. A similar strategy can be used to measure other biomarkers such as Her-3, p-95 and the like.

In certain embodiments, activating the cleavage-inducing moiety cleaves the cleavable linker. In certain embodiments, the binding compound specifically binds a Her-2 or Her-3 epitope. In certain embodiments, the binding compound comprises an antibody or antigen-binding fragment. In certain embodiments, the binding compound specifically binds a Her-2 ligand binding site or a Her-3 ligand binding site. In certain embodiments, the binding compound comprises a Her-2 ligand and/or a Her-3 ligand. In certain embodiments, the binding compound and the cleaving probe bind the same Her-2 epitope and/or a Her-3 epitope.

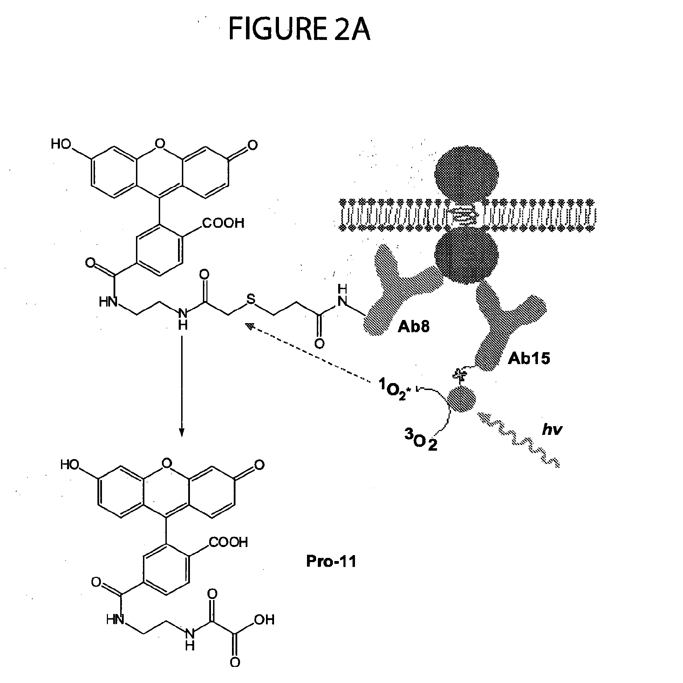

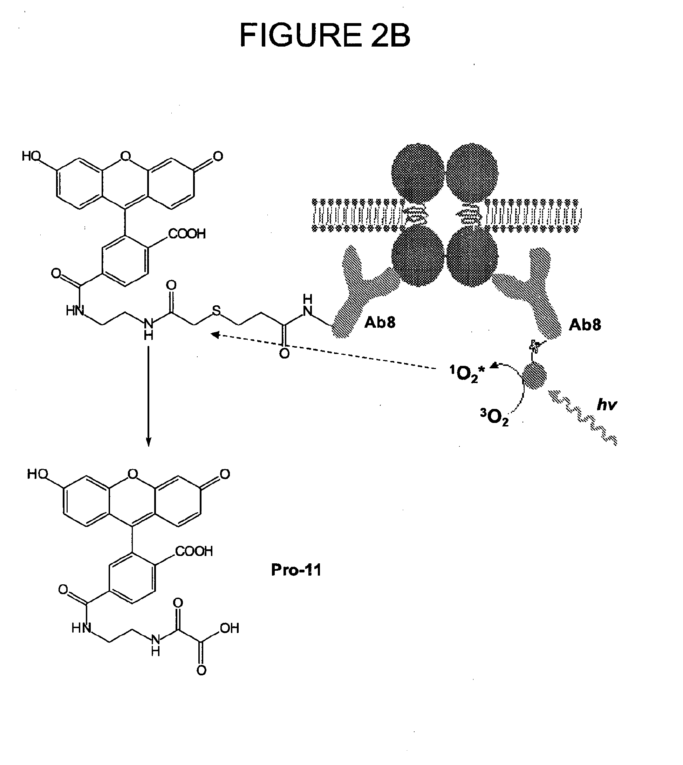

In certain embodiments, and as illustrated in Figure 2A showing measurement of Her-2 total using two different antibodies, one with a cleaving agent and one with a tag, and Figure 2B showing measurement of Her-2 dimers using a single antibody alternately attached to either a cleaving agent or a binding moiety, the step of measuring the amounts of one or more Her-2 homodimers, Her-3 homodimers, or Her-2/Her-3 heterodimers comprises the following steps: (i) providing for each of the one or more Her-2 homodimers a cleaving probe (e.g., antibody 15 in Figure 2A, and antibody 8 in Figure 2B) specific for a first Her-2 protein in each of the one Her-2 homodimers, each cleaving probe having a cleavage-inducing moiety with an effective proximity; (ii) providing one or more binding compounds (e.g., antibody 8 in both Figures 2A and 2B) specific for a second protein of each of the one or more Her-2 homodimers, such that each binding compound has one or more molecular tags each attached thereto by a cleavable linkage, and such that the one or more molecular tags attached to different binding compounds have different separation characteristics so that upon separation molecular tags from different binding compounds form distinct peaks in a separation profile; (iii) mixing the cleaving probes, the binding compounds, and the one or more complexes such that cleaving probes specifically bind to first proteins of the Her-2 homodimers and binding compounds specifically bind to the second proteins of the Her-2 homodimers and such that cleavable linkages of the binding compounds are within the effective proximity of cleavage-inducing moieties of the cleaving probes so that molecular tags are released; and (iv) separating and identifying the released molecular tags to determine the presence or absence or the amount of the Her-2 homodimers.

In certain embodiments, the step of measuring the amounts of one or more Her-2 homodimers, Her-3 homodimers, or Her-2/Her-3 heterodimers comprises the following steps: (i) providing for each of the one or more Her-2 homodimers a cleaving probe specific for a first Her-2 protein in each of the one Her-2 homodimers, each cleaving probe having a cleavage-inducing moiety with an effective proximity; (ii) providing one or more binding compounds specific for a second protein of each of the one or more Her-3 homodimers, such that each binding compound has one or more molecular tags each attached thereto by a cleavable linkage, and such that the one or more molecular tags attached to different binding compounds have different separation characteristics so that upon separation molecular tags from different binding compounds form distinct peaks in a separation profile; (iii) mixing the cleaving probes, the binding compounds, and the one or more complexes such that cleaving probes specifically bind to first proteins of the Her-3 homodimers and binding compounds specifically bind to the second proteins of the Her-3 homodimers and such that cleavable linkages of the binding compounds are within the effective proximity of cleavage-inducing moieties of the cleaving probes so that molecular tags are released; and (iv) separating and identifying the released molecular tags to determine the presence or absence or the amount of the Her-3 homodimers.

The Her-2/Her-3 heterodimers can be similarly determined using cleaving probes and binding probes specific to Her-2 and Her-3, e.g., a cleaving probe specific to Her-2 and a binding probe specific to Her-3 and/or a cleaving probe specific to Her-3 and a binding probe specific to Her-2.

The disclosure relates to Her-2-acting agents. A Her-2-acting agent can be any such agent known to one of skill in the art. In certain embodiments the Her2-acting agent is selected from the group consisting of 4D5; trastuzumab, AEE-788 and lapatinib. In a preferred embodiment, the Her-2-acting agent is trastuzumab (Herceptin^{®}). *See, e.g.,* Goldenberg, 1999, Clin Ther. 21:309-18; and Shak, 1999, Semin Oncol. 26:71-7. Also, other Her-2 acting agents may be evaluated using the methods described herein.

Samples containing Her-2 and/or Her-2 homodimers and/or Her-3 and/or Her-3 homodimers and/or Her-2/Her-3 heterodimers suitable for use as biomarkers may come from a wide variety of sources, including cell cultures, animal or plant tissues, patient biopsies or the like. Preferably, samples are human patient samples. Samples are prepared for assays of the invention using conventional techniques, which may depend on the source from which a sample is taken. For biopsies and medical specimens, guidance is provided in the following references: Bancroft JD & Stevens A, eds. 1977, Theory and Practice of Histological Techniques; Churchill Livingstone, Edinburgh,; Pearse, 1980, Histochemistry. Theory and applied. 4th ed., Churchill Livingstone, Edinburgh.

In the area of cancerous disease status, examples of patient tissue samples that may be used include, but are not limited to, breast, prostate, ovary, colon, lung, endometrium, stomach, salivary gland or pancreas. The tissue sample can be obtained by a variety of procedures including surgical excision, aspiration or biopsy. The tissue may be fresh or frozen. In one embodiment, assays of the invention are carried out on tissue samples that have been fixed and embedded in paraffin and a step of deparaffination is be carried out. A tissue sample may be fixed *(i.e.,* preserved) by conventional methodology. *See, e.g.,* Lee G. Luna, HT (ASCP) Ed:, 1960, Manual of Histological Staining Method of the Armed Forces Institute of Pathology 3rd edition, The Blakston Division McGraw-Hill Book Company, New York; Ulreka V. Mikel, Ed., 1994, The Armed Forces Institute of Pathology Advanced Laboratory Methods in Histology and Pathology, Armed Forces Institute of Pathology, American Registry of Pathology, Washington, D.C. One of skill in the art will appreciate that the choice of a fixative is determined by the purpose for which the tissue is to be histologically stained or otherwise analyzed. One of skill in the art will also appreciate that the length of fixation depends upon the size of the tissue sample and the fixative used.

Generally, a tissue sample is first fixed and is then dehydrated through an ascending series of alcohols, infiltrated and embedded with paraffin or other sectioning media so that the tissue sample may be sectioned. Alternatively, one may section the tissue and fix the sections obtained. By way of example, the tissue sample may be embedded and processed in paraffin by conventional methodology according to conventional techniques described by the references provided above. Examples of paraffin that may be used include, but are not limited to, Paraplast, Broloid, and Tissuemay. Once the tissue sample is embedded, the sample may be sectioned by a microtome according to conventional techniques. Sections may have a thickness in a range from about three microns to about twelve microns, and preferably, a thickness in a range of from about 5 microns to about 10 microns. In one aspect, a section may have an area of from about 10 mm² to about 1 cm². Once cut, the sections may be attached to slides by several standard methods. Examples of slide adhesives include, but are not limited to, silane, gelatin and poly-L-lysine. Paraffin embedded sections may be attached to positively charged slides and/or slides coated with poly-L-lysine.

If paraffin has been used as the embedding material, the tissue sections are generally deparaffinized and rehydrated to water prior to detection of biomarkers. Tissue sections may be deparaffinized by several conventional standard methodologies. For example, xylenes and a gradually descending series of alcohols may be used according to conventional techniques described by the references provided above. Alternatively, commercially available deparaffinizing non-organic agents such as Hemo-De® (CMS, Houston, Tex.) may be used.

Mammalian tissue culture cells, or fresh or frozen tissues may be prepared by conventional cell lysis techniques (e.g., 0.14 M NaCl, 1.5 mM MgCl₂, 10 mM Tris-Cl (pH 8.6), 0.5% Nonidet P-40, and protease and/or phosphatase inhibitors as required). For fresh mammalian tissues, sample preparation may also include a tissue disaggregation step, such as crushing, mincing, grinding or sonication.

Many advantages are provided by measuring dimer populations using releasable molecular tags, including (1) separation of released molecular tags from an assay mixture provides greatly reduced background and a significant gain in sensitivity; and (2) the use of molecular tags that are specially designed for ease of separation and detection provides a convenient multiplexing capability so that multiple receptor complex components may be readily measured simultaneously in the same assay. Assays employing such tags can have a variety of forms and are disclosed in the following references: U.S. Patent Numbers 7,105,308 and 6,627,400; published U.S. Patent Application Nos. 2002/0013126, 2003/0170915, 2002/0146726, and 2009/0191559; and International Patent Publication No. WO 2004/011900. For example, a wide variety of separation techniques may be employed that can distinguish molecules based on one or more physical, chemical or optical differences among molecules being separated including electrophoretic mobility, molecular weight, shape, solubility, pKa, hydrophobicity, charge, charge/mass ratio or polarity. In one aspect, molecular tags in a plurality or set differ in electrophoretic mobility and optical detection characteristics and are separated by electrophoresis. In another aspect, molecular tags in a plurality or set may differ in molecular weight, shape, solubility, pKa, hydrophobicity, charge, polarity and are separated by normal phase or reverse phase HPLC, ion exchange HPLC, capillary electrochromatography, mass spectroscopy or gas phase chromatography.

Sets of molecular tags are provided that can be separated into distinct bands or peaks by a separation technique after they are released from binding compounds. Identification and quantification of such peaks provides a measure or profile of the presence and/or amounts of receptor dimers. Molecular tags within a set may be chemically diverse; however, for convenience, sets of molecular tags are usually chemically related. For example, they may all be peptides or they may consist of different combinations of the same basic building blocks or monomers or they may be synthesized using the same basic scaffold with different substituent groups for imparting different separation characteristics. The number of molecular tags in a plurality may vary depending on several factors including the mode of separation employed, the labels used on the molecular tags for detection, the sensitivity of the binding moieties and the efficiency with which the cleavable linkages are cleaved.

Measurements made directly on tissue samples may be normalized by including measurements on cellular or tissue targets that are representative of the total cell number in the sample and/or the numbers of particular subtypes of cells in the sample. The additional measurement may be preferred, or even necessary, because of the cellular and tissue heterogeneity in patient samples, particularly tumor samples, which may comprise substantial fractions of normal cells.

As mentioned above, mixtures containing pluralities of different binding compounds may be provided, wherein each different binding compound has one or more molecular tags attached through cleavable linkages. The nature of the binding compound, cleavable linkage and molecular tag may vary widely. A binding compound may comprise an antibody binding composition, an antibody, a peptide, a peptide or non-peptide ligand for a cell surface receptor, a protein, an oligonucleotide, an oligonucleotide analog, such as a peptide nucleic acid, a lectin or any other molecular entity that is capable of specifically binding to a target protein or molecule or stable complex formation with an analyte of interest, such as a Her-2 homodimer. In one aspect, a binding compound can be represented by the following formula:

B-(L-E)ₖ

wherein B is binding moiety; L is a cleavable linkage and E is a molecular tag. In homogeneous assays, cleavable linkage, L, may be an oxidation-labile linkage, and more preferably, it is a linkage that may be cleaved by singlet oxygen. The moiety "-(L-E)ₖ" indicates that a single binding compound may have multiple molecular tags attached via cleavable linkages. In one aspect, k is an integer greater than or equal to one, but in other embodiments, k may be greater than several hundred, e.g. 100 to 500 or k is greater than several hundred to as many as several thousand, *e.g.* 500 to 5000. Usually each of the plurality of different types of binding compounds has a different molecular tag, E. Cleavable linkages, e.g. oxidation-labile linkages, and molecular tags, E, are attached to B by way of conventional chemistries.

Preferably, B is an antibody binding composition that specifically binds to a target, such as an aritigenic determinant on Her-2. Antibodies specific for Her-2 epitopes are provided in the examples set forth herein. Antibody compositions are readily formed from a wide variety of commercially available antibodies, either monoclonal or polyclonal. In particular, antibodies specific for epidermal growth factor receptors are disclosed in U.S. Patent Nos. 5,677,171; 5,772,997; 5,968,511; 5,480,968; 5,811,098, U.S. Patent 5,599,681, discloses antibodies specific for phosphorylation sites of proteins. Commercial vendors, such as Cell Signaling Technology (Beverly, MA), Biosource International (Camarillo, CA) and Upstate (Charlottesville, VA) also provide monoclonal and polyclonal antibodies.

Cleavable linkage, L, can be virtually any chemical linking group that may be cleaved under conditions that do not degrade the structure or affect detection characteristics of the released molecular tag, E. Whenever a cleaving probe is used in a homogeneous assay format, cleavable linkage, L, is cleaved by a cleavage agent generated by the cleaving probe that acts over a short distance so that only cleavable linkages in the immediate proximity of the cleaving probe are cleaved. Typically, such an agent must be activated by making a physical or chemical change to the reaction mixture so that the agent produces a short lived active species that diffuses to a cleavable linkage to effect cleavage. In a homogeneous format, the cleavage agent is preferably attached to a binding moiety, such as an antibody, that targets prior to activation the cleavage agent to a particular site in the proximity of a binding compound with releasable molecular tags. In such embodiments, a cleavage agent is referred to herein as a "cleavage-inducing moiety."

In a non-homogeneous format, because specifically bound binding compounds are separated from unbound binding compounds, a wider selection of cleavable linkages and cleavage agents are available for use. Cleavable linkages may not only include linkages that are labile to reaction with a locally acting reactive species, such as hydrogen peroxide, singlet oxygen or the like, but also linkages that are labile to agents that operate throughout a reaction mixture, such as base-labile linkages, photocleavable linkages, linkages cleavable by reduction, linkages cleaved by oxidation, acid-labile linkages and peptide linkages cleavable by specific proteases. References describing many such linkages include Greene and Wuts, 1991, Protective Groups in Organic Synthesis, Second Edition, John Wiley & Sons, New York; Hermanson, 1996, Bioconjugate Techniques, Academic Press, New York; and U.S. Patent No. 5,565,324.

In one aspect, commercially available cleavable reagent systems may be employed with the invention. For example, a disulfide linkage may be introduced between an antibody binding composition and a molecular tag using a heterofunctional agent such as N-succinimidyl 3-(2-pyridyldithio)propionate (SPDP), succinimidyloxycarbonyl-α-methyl-α-(2-pyridyldithio)toluene (SMPT) or the like, available from vendors such as Pierce Chemical Company (Rockford, IL). Disulfide bonds introduced by such linkages can be broken by treatment with a reducing agent, such as dithiothreitol (DTT), dithioerythritol (DTE), 2-mercaptoethanol or sodium borohydride. Typical concentrations of reducing agents to effect cleavage of disulfide bonds are in the range of from 10 to 100 mM. An oxidatively labile linkage may be introduced between an antibody binding composition and a molecular tag using the homobifunctional NHS ester cross-linking reagent, disuccinimidyl tartarate (DST)(available from Pierce) that contains central cis-diols that are susceptible to cleavage with sodium periodate (e.g., 15 mM periodate at physiological pH for 4 hours). Linkages that contain esterified spacer components may be cleaved with strong nucleophilic agents, such as hydroxylamine, e.g., 0.1 N hydroxylamine, pH 8.5, for 3-6 hours at 37°C. Such spacers can be introduced by a homobifunctional cross-linking agent such as ethylene glycol bis(succinimidylsuccinate)(EGS) available from Pierce (Rockford, IL). A base labile linkage can be introduced with a sulfone group. Homobifunctional cross-linking agents that can be used to introduce sulfone groups in a cleavable linkage include bis[2-(succinimidyloxycarbonyloxy)ethyl]sulfone (BSOCOES), and 4,4-difluoro-3,3-dinitrophenylsulfone (DFDNPS). Exemplary basic conditions for cleavage include 0.1 M sodium phosphate, adjusted to pH 11.6 by addition of Tris base, containing 6 M urea, 0.1% SDS, and 2 mM DTT, with incubation at 37 °C for 2 hours. Photocleavable linkages also include those disclosed in U.S. Patent No. 5,986,076.

When L is oxidation labile, L may be a thioether or its selenium analog; or an olefin, which contains carbon-carbon double bonds, wherein cleavage of a double bond to an oxo group, releases the molecular tag, E. Illustrative oxidation labile linkages are disclosed in U.S. Patent Nos. 6,627,400 and 5,622,929 and in published U.S. Patent Application Nos. 2002/0013126 and 2003/0170915.

Molecular tag, E, in the present invention may comprise an electrophoric tag as described in the following references when separation of pluralities of molecular tags are carried out by gas chromatography or mass spectrometry: *See, e.g.,* Zhang et al., 2002, Bioconjugate Chem. 13:1002-1012; Giese, 1983, Anal. Chem. 2:165-168; and U.S. Patent Nos. 4,650,750; 5,360,819; 5,516,931; and 5,602,273.

Molecular tag, E, is preferably a water-soluble organic compound that is stable with respect to the active species, especially singlet oxygen, and that includes a detection or reporter group. Otherwise, E may vary widely in size and structure. In one aspect, E has a molecular weight in the range of from about 50 to about 2500 daltons, more preferably, from about 50 to about 1500 daltons. E may comprise a detection group for generating an electrochemical, fluorescent or chromogenic signal. In embodiments employing detection by mass, E may not have a separate moiety for detection purposes. Preferably, the detection group generates a fluorescent signal.

Molecular tags within a plurality are selected so that each has a unique separation characteristic and/or a unique optical property with respect to the other members of the same plurality. In one aspect, the chromatographic or electrophoretic separation characteristic is retention time under a set of standard separation conditions conventional in the art, *e.g.,* voltage, column pressure, column type, mobile phase or electrophoretic separation medium. In another aspect, the optical property is a fluorescence property, such as emission spectrum, fluorescence lifetime or fluorescence intensity at a given wavelength or band of wavelengths. Preferably, the fluorescence property is fluorescence intensity. For example, each molecular tag of a plurality may have the same fluorescent emission properties, but each will differ from one another by virtue of a unique retention time. On the other hand, one or two or more of the molecular tags of a plurality may have identical migration or retention times, but they will have unique fluorescent properties, e.g. spectrally resolvable emission spectra, so that all the members of the plurality are distinguishable by the combination of molecular separation and fluorescence measurement.

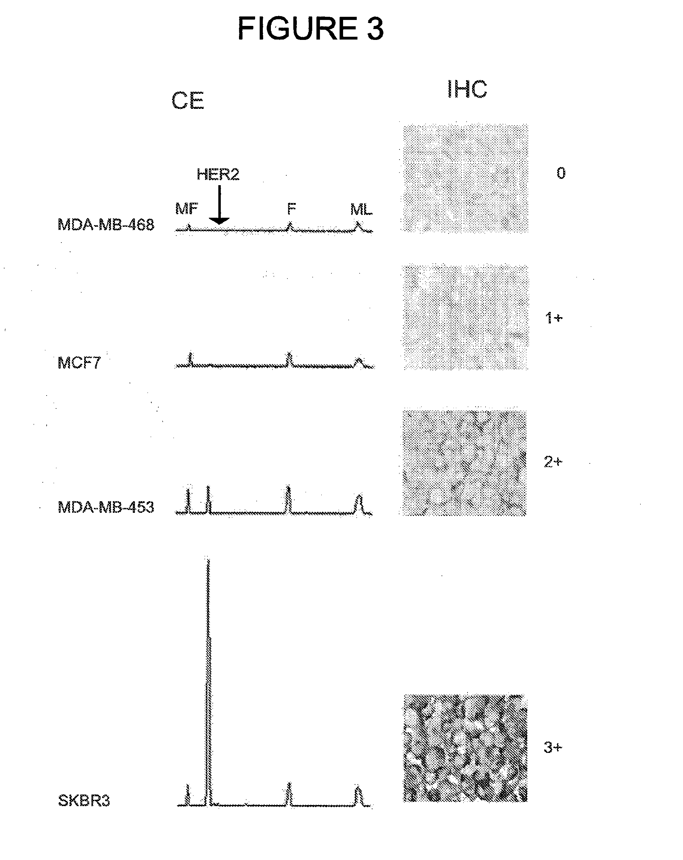

Preferably, released molecular tags are detected by electrophoretic separation and the fluorescence of a detection group. In such embodiments, molecular tags having substantially identical fluorescence properties have different electrophoretic mobilities so that distinct peaks in an electropherogram are formed under separation conditions. Preferably, pluralities of molecular tags of the invention are separated by conventional capillary electrophoresis apparatus, either in the presence or absence of a conventional sieving matrix. During or after electrophoretic separation, the molecular tags are detected or identified by recording fluorescence signals and migration times (or migration distances) of the separated compounds or by constructing a chart of relative fluorescent and order of migration of the molecular tags *(e.g.,* as an electropherogram). Preferably, the presence, absence and/or amounts of molecular tags are measured by using one or more standards as disclosed by published U.S. Patent Application No. 2003/0170734A1, Figure 3 shows an example of electrophoretic separation of a Her-2 tag in accordance with an embodiment of the present invention.

Pluralities of molecular tags may also be designed for separation by chromatography based on one or more physical characteristics that include molecular weight, shape, solubility, pKa, hydrophobicity, charge, polarity or the like, *e.g.* as disclosed in published U.S. Patent Application No. 2003/0235832. A chromatographic separation technique is selected based on parameters such as column type, solid phase, mobile phase and the like, followed by selection of a plurality of molecular tags that may be separated to form distinct peaks or bands in a single operation. Several factors determine which HPLC technique is selected for use in the invention, including the number of molecular tags to be detected (i.e., the size of the plurality), the estimated quantities of each molecular tag that will be generated in the assays, the availability and ease of synthesizing molecular tags that are candidates for a set to be used in multiplexed assays, the detection modality employed and the availability, robustness, cost and ease of operation of HPLC instrumentation, columns and solvents. Generally, columns and techniques are favored that are suitable for analyzing limited amounts of sample and that provide the highest resolution separations. Guidance for making such selections can be found in the literature, such as, for example, Snyder et al., 1988, Practical HPLC Method Development, John Wiley & Sons, New York; Millner, 1999, High Resolution Chromatography: A Practical Approach, Oxford University Press, New York; Chi-San Wu, 1999, Column Handbook for Size Exclusion Chromatography, Academic Press, San Diego; and Oliver, 1989, HPLC of Macromolecules: A Practical Approach, Oxford University Press, Oxford, England.

In one aspect, molecular tag, E, is (M, D), where M is a mobility-modifying moiety and D is a detection moiety. The notation "(M, D)" is used to indicate that the ordering of the M and D moieties may be such that either moiety can be adjacent to the cleavable linkage, L. That is, "B-L-(M, D)" designates binding compound of either of two forms: "B-L-M-D" or "B-L-D-M."

Detection moiety, D, may be a fluorescent label or dye, a chromogenic label or dye or an electrochemical label. Preferably, D is a fluorescent dye. Exemplary fluorescent dyes for use with the invention include water-soluble rhodamine dyes, fluoresceins, 4,7-dichlorofluoresceins, benzoxanthene dyes and energy transfer dyes, as disclosed in the following references: Anonymous, 2002, Handbook of Molecular Probes and Research Reagents, 8th ed., Molecular Probes, Eugene, OR; U.S. Patent Nos. 6,191,278, 6,372,907, 6,096,723, 5,945,526, 4,997,928, and 4,318,846; and Lee et al., 1997, Nucleic Acids Research 25:2816-2822. Preferably, D is a fluorescein or a fluorescein derivative.

Once each of the binding compounds is separately derivatized by a different molecular tag, it is pooled with other binding compounds to form a plurality of binding compounds. Usually, each different kind of binding compound is present in a composition in the same proportion; however, proportions may be varied as a design choice so that one or a subset of particular binding compounds are present in greater or lower proportion depending on the desirability or requirements for a particular embodiment or assay: Factors that may affect such design choices include, but are not limited to, antibody affinity and avidity for a particular target, relative prevalence of a target, fluorescent characteristics of a detection moiety of a molecular tag and the like.

A cleavage-inducing moiety, or cleaving agent, is a group that produces an active species that is capable of cleaving a cleavable linkage, preferably by oxidation. Preferably, the active species is a chemical species that exhibits short-lived activity so that its cleavage-inducing effects are only in the proximity of the site of its generation. Either the active species is inherently short lived, so that it will not create significant background beyond the proximity of its creation, or a scavenger is employed that efficiently scavenges the active species, so that it is not available to react with cleavable linkages beyond a short distance from the site of its generation. Illustrative active species include singlet oxygen, hydrogen peroxide, NADH, and hydroxyl radicals, phenoxy radical, superoxide and the like. Illustrative quenchers for active species that cause oxidation include polyenes, carotenoids, vitamin E, vitamin C, amino acid-pyrrole N-conjugates of tyrosine, histidine and glutathione. *See, e.g.* Beutner et al., 2000, Meth. Enzymol. 319:226-241.

One consideration in designing assays employing a cleavage-inducing moiety and a cleavable linkage is that they not be so far removed from one another when bound to a receptor complex that the active species generated by the cleavage-inducing moiety cannot efficiently cleave the cleavable linkage. In one aspect, cleavable linkages preferably are within about 1000 nm and preferably within about 20-200 nm, of a bound cleavage-inducing moiety. More preferably, for photosensitizer cleavage-inducing moieties generating singlet oxygen, cleavable linkages are within about 20-100 nm of a photosensitizer in a receptor complex. The range within which a cleavage-inducing moiety can effectively cleave a cleavable linkage (that is, cleave enough molecular tag to generate a detectable signal) is referred to herein as its "effective proximity." One of ordinary skill in the art will recognize that the effective proximity of a particular sensitizer may depend on the details of a particular assay design and may be determined or modified by routine experimentation.

A sensitizer is a compound that can be induced to generate a reactive intermediate, or species, usually singlet oxygen. Preferably, a sensitizer used in accordance with the invention is a photosensitizer. Other sensitizers included within the scope of the invention are compounds that on excitation by heat, light, ionizing radiation or chemical activation will release a molecule of singlet oxygen. The best known members of this class of compounds include the endoperoxides such as 1,4-biscarboxyethyl-1,4-naphthalene endoperoxide, 9,10-diphenylanthracene-9,10-endoperoxide and 5,6,11,12-tetraphenyl naphthalene 5,12-endoperoxide. Heating or direct absorption of light by these compounds releases singlet oxygen. Further sensitizers are disclosed by Di Mascio et al., 1994, FEBS Lett. 355:287; and Kanofsky, 1983, J.Biol. Chem. 258:5991-5993; Pierlot et al., 2000, Meth. Enzymol. 319:3-20.

Photosensitizers may be attached directly or indirectly, via covalent or non-covalent linkages, to the binding agent of a class-specific reagent. Guidance for constructing such compositions, particularly for antibodies as binding agents are available in the literature, *e.g.* in the fields of photodynamic therapy, immunodiagnostics, and the like. Exemplary guidance may be found in Ullman et al., 1994, Proc. Natl. Acad. Sci. USA 91, 5426-5430; Strong et al., 1994, Ann. New York Acad. Sci. 745: 297-320; Yarmush et al., 1993, Crit. Rev. Therapeutic Drug Carrier Syst. 10: 197-252; and U.S. Patent Nos. 5,709,994, 5,340,716, 6,251,581, and 5,516,636.

A large variety of light sources are available to photo-activate photosensitizers to generate singlet oxygen. Both polychromatic and monochromatic sources may be used as long as the source is sufficiently intense to produce enough singlet oxygen in a practical time duration. The length of the irradiation depends on the nature of the photosensitizer, the nature of the cleavable linkage, the power of the source of irradiation and its distance from the sample. In general, the period for irradiation may be less than about a microsecond to as long as about 10 minutes, usually in the range of about one millisecond to about 60 seconds. The intensity and length of irradiation should be sufficient to excite at least about 0.1% of the photosensitizer molecules, usually at least about 30% of the photosensitizer molecules and preferably, substantially all of the photosensitizer molecules. Exemplary light sources include lasers such as, e.g., helium-neon lasers, argon lasers, YAG lasers, He/Cd lasers and ruby lasers; photodiodes; mercury, sodium and xenon vapor lamps; incandescent lamps such as, e.g., tungsten and tungsten/halogen and flashlamps. An exemplary photoactivation device suitable for use in the methods of the invention is disclosed International Patent Publication No. WO 03/051669. In such embodiments, the photoactivation device is an array of light emitting diodes (LEDs) mounted in housing that permits the simultaneous illumination of all the wells in a 96-well plate.

Examples of photosensitizers that may be utilized in the present invention are those that have the above properties and those disclosed by U.S. Patent Nos. 5,536,834, 5,763,602, 5,565,552, 5,709,994, 5,340,716, 5,516,636, 6,251,581, and 6,001,673; published European Patent Application No. 0484027; Martin et al., 1990, Methods Enzymol. 186:635-645; and Yarmush et al., 1993, Crit. Rev. Therapeutic Drug Carrier Syst. 10:197-252. As with sensitizers, in certain embodiments, a photosensitizer,may be associated with a solid phase support by being covalently or non-covalently attached to the surface of the support or incorporated into the body of the support. In general, the photosensitizer is associated with the support in an amount necessary to achieve the necessary amount of singlet oxygen. Generally, the amount of photosensitizer is determined empirically according to routine methods.

In one embodiment, a photosensitizer is incorporated into a latex particle to form photosensitizer beads, *e.g.* as disclosed by U.S. Patent Nos. 5,709,994 and 6,346,384; and International Patent Publication No. WO 01/84157. Alternatively, photosensitizer beads may be prepared by covalently attaching a photosensitizer, such as rose bengal, to 0.5 micron latex beads by means of chloromethyl groups on the latex to provide an ester linking group, as described in J. Amer. Chem. Soc., 97:3741 (1975). This reaction may be carried out, for example, in a conventional 96-well or 384-well microtiter plate, or the like, having a filter membrane that forms one wall, *e.g.* the bottom, of the wells that allows reagents to be removed by the application of a vacuum. This allows the convenient exchange of buffers, if the buffer required for specific binding of binding compounds is different than the buffer required for either singlet oxygen generation or separation. For example, in the case of antibody-based binding compounds, a high salt buffer is required. If electrophoretic separation of the released tags is employed, then better performance is achieved by exchanging the buffer for one that has a lower salt concentration suitable for electrophoresis.

As an example, a cleaving probe may comprise a primary haptenated antibody and a secondary anti-hapten binding protein derivatized with multiple photosensitizer molecules. A preferred primary haptenated antibody is a biotinylated antibody and preferred secondary anti-hapten binding proteins may be either an anti-biotin antibody or streptavidin. Other combinations of such primary and secondary reagents are well known in the art. Exemplary combinations of such reagents are taught by Haugland, 2002, Handbook of Fluorescent Probes and Research Reagents, Ninth Edition, Molecular Probes, Eugene, OR. An exemplary combination of such reagents is described below. There binding compounds having releasable tags ("mT₁" and "mT₂"), and primary antibody derivatized with biotin are specifically bound to different epitopes of receptor dimer in membrane. Biotin-specific binding protein, e.g. streptavidin, is attached to biotin bringing multiple photosensitizers into effective proximity of binding compounds. Biotin-specific binding protein may also be an anti-biotin antibody and photosensitizers may be attached via free amine group on the protein by conventional coupling chemistries, e.g., Hermanson (supra). An exemplary photosensitizer for such use is an NHS ester of methylene blue prepared as disclosed in published European Patent Application 0510688.

The following general discussion of methods and specific conditions and materials are by way of illustration and not limitation. One of skill in the art will understand how the methods described herein can be adapted to other applications, particularly with using different samples, cell types and target complexes.

In conducting the methods of the invention, a combination of the assay components is made, including the sample being tested, the binding compounds and optionally the cleaving probe. Generally, assay components may be combined in any order. In certain applications, however, the order of addition may be relevant. For example, one may wish to monitor competitive binding, such as in a quantitative assay. Or one may wish to monitor the stability of an assembled complex. In such applications, reactions may be assembled in stages.

The amounts of each reagent can generally be determined empirically. The amount of sample used in an assay will be determined by the predicted number of target complexes present and the means of separation and detection used to monitor the signal of the assay. In general, the amounts of the binding compounds and the cleaving probe can be provided in molar excess relative to the expected amount of the target molecules in the sample, generally at a molar excess of at least about 1.5, more desirably about 10-fold excess, or more. In specific applications, the concentration used may be higher or lower, depending on the affinity of the binding agents and the expected number of target molecules present on a single cell. Where one is determining the effect of a chemical compound on formation of oligomeric cell surface complexes, the compound may be added to the cells prior to, simultaneously with or after addition of the probes, depending on the effect being monitored.

The assay mixture can be combined and incubated under conditions that provide for binding of the probes to the cell surface molecules, usually in an aqueous medium, generally at a physiological pH (comparable to the pH at which the cells are cultures), maintained by a buffer at a concentration in the range of about 10 to 200 mM. Conventional buffers may be used, as well as other conventional additives as necessary, such as salts, growth medium, stabilizers, etc. Physiological and constant temperatures are normally employed. Incubation temperatures normally range from about 4° to 70°C, usually from about 15° to 45°C, more usually about 25° to 37°C.

After assembly of the assay mixture and incubation to allow the probes to bind to cell surface molecules, the mixture can be treated to activate the cleaving agent to cleave the tags from the binding compounds that are within the effective proximity of the cleaving agent, releasing the corresponding tag from the cell surface into solution. The nature of this treatment will depend on the mechanism of action of the cleaving agent. For example, where a photosensitizer is employed as the cleaving agent, activation of cleavage can comprise irradiation of the mixture at the wavelength of light appropriate to the particular sensitizer used.

Following cleavage, the sample can then be analyzed to determine the identity of tags that have been released. Where an assay employing a plurality of binding compounds is employed, separation of the released tags will generally precede their detection. The methods for both separation and detection are determined in the process of designing the tags for the assay. A preferred mode of separation employs electrophoresis, in which the various tags are separated based on known differences in their electrophoretic mobilities.

As mentioned above, in some embodiments, if the assay reaction conditions may interfere with the separation technique employed, it may be necessary to remove, or exchange, the assay reaction buffer prior to cleavage and separation of the molecular tags. For example, assay conditions may include salt concentrations (e.g. required for specific binding) that degrade separation performance when molecular tags are separated on the basis of electrophoretic mobility. Thus, such high salt buffers may be removed, e.g., prior to cleavage of molecular tags, and replaced with another buffer suitable for electrophoretic separation through filtration, aspiration, dilution or other means.

In certain embodiments, the subject may be administered a combination therapy that includes trastuzumab. The combination therapy can include trastuzumab in combination with one or more of any chemotherapeutic agent known to one of skill in the art without limitation. Preferably, the chemotherapeutic agent has a different mechanism of action from trastuzumab. For example, the chemotherapeutic agent can be an anti-metabolite (e.g., 5-flourouricil (5-FU), methotrexate (MTX), fludarabine, etc.), an antimicrotubule agent (e.g., vincristine; vinblastine; taxanes such as paclitaxel and docetaxel; etc.), an alkylating agent (*e.g.,* cyclophosphamide, melphalan, bischloroethylnitrosurea, etc.), platinum agents (*e.g.,* cisplatin, carboplatin, oxaliplatin, JM-216, CI-973, etc.), anthracyclines (e.g., doxorubicin, daunorubicin, etc.), antibiotic agents (*e.g.,* mitomycin-C, actinomycin D, etc.), topoisomerase inhibitors (e.g., etoposide, camptothecins, etc.) or other any other chemotherapeutic agents known to one skilled in the art.

Particular examples of chemotherapeutic agents that can be used in the various embodiments of the invention, including pharmaceutical compositions, dosage forms, and kits of the invention, include, without limitation, cytarabine, melphalan, topotecan, fludarabine, etoposide, idarubicin, daunorubicin, mitoxantrone, cisplatin paclitaxel, and cyclophosphamide.

Other chemotherapeutic agents that may be used include abarelix, aldesleukin, alemtuzumab, alitretinoin, allopurinol, altretamine, amifostine, anastrozole, arsenic trioxide, asparaginase, BCG live, bevaceizumab, bexarotene, bleomycin, bortezomib, busulfan, calusterone, camptothecin, capecitabine, carboplatin, carmustine, celecoxib, cetuximab, chlorambucil, cinacalcet, cisplatin, cladribine, cyclophosphamide, cytarabine, dacarbazine, dactinomycin, darbepoetin alfa, daunorubicin, denileukin diftitox, dexrazoxane, docetaxel, doxorubicin, dromostanolone, Elliott's B solution, epirubicin, epoetin alfa, estramustine, etoposide, exemestane,filgrastim, floxuridine, fludarabine, fluorouracil, fulvestrant, gemcitabine, gemtuzumab ozogamicin, gefitinib, goserelin, hydroxyurea, ibritumomab tiuxetan, idarubicin, ifosfamide, imatinib, interferon alfa-2a, interferon alfa-2b, irinotecan, letrozole, leucovorin, levamisole, lomustine, meclorethamine, megestrol, melphalan, mercaptopurine, mesna, methotrexate, methoxsalen, methylprednisolone, mitomycin C, mitotane, mitoxantrone, nandrolone, nofetumomab, oblimersen, oprelvekin, oxaliplatin, paclitaxel, pamidronate, pegademase, pegaspargase, pegfilgrastim, pemetrexed, pentostatin, pipobroman, plicamycin, polifeprosan, porfimer, procarbazine, quinacrine, rasburicase, rituximab, sargramostim, streptozocin, talc, tamoxifen, tarceva, temozolomide, teniposide, testolactone, thioguanine, thiotepa, topotecan, toremifene, tositumomab, trastuzumab, tretinoin, uracil mustard, valrubicin, vinblastine, vincristine, vinorelbine, and zoledronate.

In another aspect, the disclosure is drawn to a method for determining whether a subject with a Her-2 positive cancer is unlikely to respond to treatment with at least one chemotherapeutic agent in addition to a Her2-acting agent and/or the patient is likely to have a short time course. In certain embodiments, the method comprises measuring in a biological sample from the subject's cancer an amount of Her-2 and/or Her-2 homodimers, wherein if the level of Her-2 and/or Her-2 homodimers is high or very high, then the patient is unlikely to respond to at least one chemotherapeutic agent in addition to a Her-2 acting agent.

In certain embodiments, may comprise stratifying the patients with high Her-2 into two groups: very high and moderately high. The stratification may comprise the use of Her-2 levels as described herein. In some embodiments the method may further comprise detecting in a biological sample from the subject's cancer the amount of Her-2 and/or Her-2 homodimers wherein if the amount of Her-2 and/or Her-2 homodimers is moderately high, then the patient group is further sub-divided (i.e., stratified) into high Her-3 expressors and low Her-3 expressors. In some embodiments, the patient with moderately high (i.e., medium) Her-2 and/or Her-2 homodimers and high Her-3 is less likely to respond to the Her-2 acting agent and/or the patient has a long time course than a patient with moderately high (i.e., medium) Her-2 and/or Her-2 homodimers and low Her-3. In certain embodiments of each of the methods of the present invention, high Her-2 expression is a log10H2T ≥ about 1.14-1.125. In certain embodiments of each of the methods disclosed herein, the high Her-2 expression comprises expression that is very high and/or moderately high. In certain embodiments of each of the methods disclosed herein, the very high Her-2 expression is a log10H2T ≥ about 1.84 -2.21. In certain embodiments of each of the methods disclosed herein, the moderately high expression is between 1.14 - 1.25 and 1.84-2.21. Or, other ranges may be used depending upon the patient cohort and/or the significant event being monitored.

In certain embodiments, the biological sample comprises FFPEs. In certain embodiments, the subject's cancer is breast cancer. In certain embodiments, the breast cancer is metastatic. In some embodiments, the breast cancer is early stage (i.e., adjuvant) breast cancer. Or, any cancer that may be sensistive to a Her-2 acting agent may be monitored. The Her-2 acting agent may be any Her-2 acting agent. In certain embodiments, the Her-2 acting agent is one of the agents described herein. For example, in certain embodiments, the Her-2 acting agent is trastuzumab. In certain embodiments, the chemotherapeutic agent is paclitaxel.

In certain embodiments, an amount of Her-2 is measured. In certain embodiments, an amount of Her-2 homodimers is measured. In certain embodiments, if the Her-2 is medium, then an amount of Her-3 is measured. In certain embodiments, if the Her-2 is moderately high (i.e. medium), then an amount of Her-3 homodimers and/or Her-2/Her-3 heterodimers is measured. In certain embodiments, the amount of Her-2 and/or her-3 is measured using an assay capable of measuring and/or quantifying an amount of protein-protein interactions in a sample. In a certain embodiment, the assay is the VERATAG® assay. In certain embodiments, likeliness to respond is measured with respect to overall survival rate, time to progression and/or using the RECIST criteria or other response criteria.

In another aspect, the disclosure is drawn to a method for determining whether a subject with a Her-2 positive cancer is likely to respond to treatment with at least one chemotherapeutic agent in addition to a Her2-acting agent. In certain embodiments, the method comprises measuring in a biological sample from the subject's cancer an amount of Her-2 and/or Her-2 homodimers, wherein if the level of Her-2 and/or Her-2 homodimers is low, then the patient is likely to respond to at least one chemotherapeutic agent in addition to the Her-2 acting agent. In certain embodiments, the biological sample comprises FFPEs. In certain embodiments, the subject's cancer is breast cancer. In certain embodiments, the breast cancer is metastatic. In some embodiments, the breast cancer is early stage (i.e., adjuvant) breast cancer. Or, any cancer that may be sensitive to a Her-2 acting agent may be monitored. The Her-2 acting agent may be any Her-2 acting agent. In certain embodiments, the Her-2 acting agent is one of the agents described herein. For example, in certain embodiments, the Her-2 acting agent is trastuzumab. In certain embodiments, the additional chemotherapeutic agent is paclitaxel. Or, other additional chemotherapeutic agents as known in the art and/or disclosed herein may be evaluated. In certain embodiments, likeliness to respond or time course is measured with respect to overall survival rate, time to progression and/or using the RECIST criteria.

In another aspect, the disclosure is drawn to a method for determining whether a subject with a Her-2 positive cancer is likely to respond to a Her-2 acting agent and/or predicting whether the time course of the disease is long and/or predicting whether the subject will have a significant event, the method comprising detecting in a biological sample from the subject's cancer the amount of Her-2 and Her-2 homodimers and determining the ratio of Her-2 homodimers to total Her-2, wherein the subject's ratio is determined to be in one of at least 3 subgroups and if the subject's ratio is in the low, or high subgroup, then the subject is likely to respond to the Her-2 acting agent, the subject is likely to have a long time course and/or the subject is not likely to have a significant event. In a preferred embodiment, the at least three subgroups are determined by comparing the Her-2 homodimer to total Her-2 ratio to the hazards ratio for populations treated with versus without a Her-2 acting agent, wherein if the hazard ratio is less than 1, then the subject is more likely to respond to the Her-2 acting agent, the patient is more likely to have a long time course and/or the patient is less likely to have a significant event.

For example, the H2D/H2T ratio, Her-2 expression (H2T) and Her-2 homodimer levels (H2D) were also examined with respect to trastuzumab responsiveness in the FIN HER clinical trial, which was designed to test the effectiveness of trastuzumab in the early stage (i.e., adjuvant) setting. H2T, H2D and H2D/H2T were compared with IHC, CISH and clinical outcomes in the study. Since Her-2 positivity by IHC or FISH has been shown to correlate with adverse prognosis and improved clinical outcomes with trastuzumab, an ability to discriminate between likely responders and nonresponders with a quantitative assay, such as the HERMark assay, was anticipated. However, as shown by multivariate Cox proportional hazards analysis, neither H2T nor H2D correlated significantly with outcome. H2D/H2T, on the other hand, was independently associated with time to any recurrence (TAR) and nearly significantly associated with time to distant recurrence (TDR). Because of these findings, STEPP (subpopulation treatment effect pattern plot) analysis was performed to examine hazard ratios for treated versus control patients across the distributions of H2T, H2D and H2D/H2T. Subpopulations of 80 patients were used for these analyses. While neither H2D nor H2T identified any group of patients who did not benefit from trastuzumab, H2D/H2T was shown to discriminate between groups of patients that respond to trastuzumab and groups of patients that do not respond to trastuzumab. This latter group has intermediate H2D/H2T ratios that fall in-between a low H2D/H2T ratio group and a high H2D/H2T ratio group. While the applicants do not wish to be confined to a mechanistic theory, one possible explanation for this observation is that H2D/H2T is a measure of Her-2 activation in breast tumors and is therefore a prognostic biomarker for Her-2-positive patients in the early stage (i.e., adjuvant) setting who do not receive trastuzumab and a predictive biomarker for the degree of clinical benefit that patients experience when treated with trastuzumab in the early stage (i.e., adjuvant) setting.

In certain embodiments, the subject's cancer is breast cancer. In certain embodiments, the subject's cancer is metastatic or primary early stage (i.e., adjuvant). In certain embodiments, the Her-2 acting agent is trastuzumab. In certain embodiments, Her-2, Her-2 homodimers, Her-3, Her-3 homodimers, and Her-2/Her-3 heterodimers are detected using the VERATAG® assay. In certain embodiments, the likeliness to respond, likeliness to have a long time course and/or likeliness to have a significant event is measured as an overall survival rate, as time to progression, as time to distant recurrence and disease-free survival and/or response or clinical benefit using the RECIST criteria. In certain embodiments, whether the cancer is Her-2 positive is determined by IHC or FISH or CISH. In other embodiments, the invention is drawn to a method comprising determining whether the Her-2 homodimer to total Her-2 ratio is low, intermediate or high by comparing the Her-2 homodimer to total Her-2 ratio of the subject's cancer to optimal cutoffs. In yet further embodiments, if the ratio of Her-2 homodimers to total Her-2 is intermediate and/or the hazard ratio is equal to or greater than 1, then the patient is less likely to respond to a Her-2 acting agent and/or the patient is less likely to have a long time course and/or the patient is more likely to have a significant event.

In a further aspect, the disclosure provides methods of treating a subject with cancer. In one aspect, the methods comprise determining that the subject is afflicted with a cancer that is likely to respond to treatment with a Her2-acting agent and/or has a long time course according to a method of the invention and administering an effective amount of a Her2-acting agent to the subject as a result of said determination. In another aspect, the methods comprise determining that a subject is afflicted with a cancer that is likely to respond to treatment with a Her2-acting agent and/or has a long time course according to a method of the invention, then advising a medical professional of the treatment option of administering to the subject an effective amount of a Her2-acting agent. In another aspect, the methods comprise determining that a subject is afflicted with a cancer that has a short time course and/or that is unlikely to respond to a chemotherapeutic agent in addition to a Her-2 acting agent. These aspects each comprise each of the various embodiments (e.g., stratification by expression of Her-2 and other markers as disclosed herein; evaluation of various Her-2 acting agents and/or other chemotherapeutic agents; analysis of various cancer types). In certain embodiments, the Her2-acting agent is trastuzumab. In certain embodiments, the chemotherapeutic agent is paclitaxel. In certain embodiments, the cancer is breast cancer. In certain embodiments, the breast cancer is metastatic or primary early stage (i.e., adjuvant).

### Examples

### Example 1: Antibodies, VERATAG®-antibody, Biotin and Molecular Scissors

Monoclonal antibodies, Ab8 against cytoplasmic domain of HER2 and Ab 15 against C-terminus of HER2, were purchased from Lab Vision. VERATAG® reporters (Prol1 and Pro14) and streptavidin-conjugated methylene blue ("molecular scissors") were synthesized and purified according to protocol described previously (See, for example, above and United States Patent 7,105,308). Antibody-VERATAG® and antibody-biotin conjugates, i.e., Ab8-Proll and Ab15-biotin, were made using sulfo-NHS-LC-LC-biotin (Pierce) as linker according to manufacturer's protocol and conjugation products purified by HPLC (Agilent).

### Example 2: Cell Culture, Fixation; Processing and Paraffin Embedding

Four breast cancer cell lines, MDA-MB-468, MCF-7, MDA-MB-453 and SKBR-3, were purchased from American Type Cell Culture Collection. All cell-lines were maintained at 37°C and 5% CO₂ in Dulbecco's modified Eagle medium (DMEM): F12 (50:50), 10% FBS, 1% PSQ (10% fetal bovine serum, 1% penicillin-streptomycin) and 2mM L-glutamine. Cells were grown to near confluence on at least ten 150-mm culture plates for each cell line. After removal of medium, the cells were washed once with cold 1xPBS and 15 mL of 10% NBF (neutral buffered formalin) was added to each plate. Cells were fixed over night (>16 hrs) at 4°C. After removal of the fixative solution, the cells were harvested by scraping with residual fixative solution and centrifuged at 3200xg for 15 min. The cell pellet was transferred to a rubber O-ring, wrapped with filter paper and placed in a processing cassette. Automatic Tissue-Tek processor was used for processing. Briefly, cell pellet was exposed to increasing concentrations of alcohol, Clear-rite (xylene substitute) and paraffin. After processing, pellet was embedded in a block using a paraffin embedding station. All solvents used for cell pellet processing were obtained from Richard-Allen Scientific.

### Example 3: Breast Tissues, Fixation, Processing and Paraffin Embedding

Frozen breast tissues with different Her-2 expression levels were purchased from Biooptions. The tissue chunks (0.9-1.9 grams) were fixed in 10% NBF for ∼24 hrs at 4°C, and processed and paraffin-embedded as described for cell line pellets.

### Example 4: Microtomy

Sections of 7 um in thickness were sliced with a microtome (LEICA) and placed on positively charged glass slides (VWR) with serial number labeled. Slides were air-dried for 30 min and then baked in a heated oven set at 60°C for 1 hr. All sample slides were stored at 4°C for future assay.

### Example 5: Immunohistochemistry and H&E Staining

Immunohistochemistry for Her-2 was performed on Ventana Discovery XT system according to manufacturer's instructions. Primary antibody against Her-2 (CB 11) and other reagents were purchased from Ventana. H&E staining of FFPE breast tissues was conducted according to standard protocol.

### Example 6: Her-2 VERATAG® Assay in Formalin Fixed, Paraffin Embedded Cell Lines and Breast Tissue

FFPE samples were deparaffinized/rehydrated using a series of solvents. Briefly, slides were sequentially soaked in xylene (2x, 5 min), 100% ethanol (2x, 5 min), 70% ethanol (2x, 5 min) and deionized water (2x, 5 min). Heat-induced epitope retrieval of the rehydrated samples was performed in a dish containing 250 mL of 1x citrate buffer (pH 6.0) (Lab Vision) using microwave oven (Spacemaker II, GE): 3 min at power 10 followed by 10 min at power 3. After being cooled down for 20 min at room temperature, the slides were rinsed once with deionized water. A hydrophobic circle was drawn on slide using a hydrophobic pen (Zymed) to retain reagents on slides. The samples were then blocked for lhr with blocking buffer that contains 1% mouse serum, 1.5% BSA and a cocktail of protease and phosphatase inhibitors (Roche) in 1 xPBS. After removal of the blocking buffer with aspiration, a mixture of VERATAG®- and biotin-conjugated antibodies (both at concentration of 4ug/mL) prepared in blocking buffer was added and binding reactions were incubated overnight in a humidified chamber at 4°C with shaking. The antibody mix was aspirated and samples were washed with wash buffer containing 0.25% TritonX-100 in 1xPBS and streptavidin-conjugated methylene blue at concentration of 2.5ug/mL in 1 xPBS was added. The concentrations of the antibody and streptavidin-photosensitizer conjugates were all optimized based on signal specificity and assay readout dynamic range using both cell line and breast tissue samples. After 1 hr incubation at room temperature, the streptavidinmethylene blue reagent was aspirated and the samples were washed in wash buffer once followed by 3 changes of deionized water. Illumination buffer containing 3 pM fluorescein and two CE internal markers (MF and ML) in 0.01 xPBS was added on sample sections. The bound VERATAG® was released at ~4°C by photo-activated cleavage using an in-house LED array illuminator equipped with an electronic ice cube (Torrey Pine Scientific). The CE sample containing the released VERATAG® reporters was collected from above the tissue section on the slides and the released VERATAG® reporters in the CE samples were separated and detected on ABI3100 CE instrument (22-cm capillary array) (Applied Biosystems) under CE injection condition of 6kV and 50 sec at 30°C.

### Example 7: Data Analysis

The identification and quantification of VERATAG® was carried out using VERATAG® Informer software (*see,* for example, United States publication number 2007/0203408-A1, which is incorporated by reference herein, including any drawings). To analyze the VERATAG® signals in a raw CE electropherogram, two CE internal markers, MF (first marker) and ML (last marker), were used to identify the VERATAG® peaks according to their electrophoretic mobility or migration time, t, relative to the two markers, i.e., [t(VERATAG®)-t(MF)]/[t(ML)-t(MF)]. The identified VERATAG® peaks were then quantified by peak area calculation for each VERATAG®. To correct for variability in VERATAG® recovery from the tissue section, and the run variability in CE injection efficiency and/or detection sensitivity across capillary array, fluorescein (3 pM) was included in the illumination and VERATAG® recovery buffer, and co-electrophoresed as an internal reference control in each sample run. The area of each VERATAG® peak is then reported as RFU or RPA by area normalization of the VERATAG® peak (VERATAG® peak area) to the internal fluorescein peak (fluorescein peak area/1 pM) and having units of concentration (pM). The final quantification terms for the target protein detected by the VERATAG® assay can be either RPA (pM) for similar samples or the RPA*IB vol/TA for variable tumor samples (=Relative peak area multiplied by the illumination buffer volume (IB) loaded onto sample section; divided by the tumor area in mm² (RPA*IB vol/TA = pmole/L*L/mm² = pmole/mm²).

### Example 8: Titration of Sample Section Size and Estimation of Tumor Area

To evaluate the ability of the VERATAG® assay to quantify the target proteins in the same sample specimen, section size of the cell line samples cut at 7 µm on slides was titrated serially using a razor blade and different numbers of microtome-cut sections of breast tissues were captured on one slide for each titration of the tissue material. After the VERATAG® assay, the cell line slides were air-dried and photo-scanned. Section area of the samples in mm² was measured and calculated on the scanned images using ImageJ software. For breast tissue sample, post-VERATAG® assay slides were H&E stained and mounted with a mounting medium (Richard-Allan Scientific). The tumor content of the tissue samples was defined by a certified pathologist using a pen marker and area of the tumor content in mm² was measured and calculated with the ImageJ software in the same manner as for the cell line samples.

### Example 9: Development of VERATAG® Assay for FFPE Cells

An outline of the FFPE VERATAG® assay is shown in Figure 1. Before the start of the assay, FFPE microtome sections were generated from human breast cancer cell lines or tumor tissues and baked onto glass slides as described above. The FFPE cell line or tumor tissue sections were deparaffinized and rehydrated by standard xylene/ethanol/water protocols, then subjected to heat-induced antigen retrieval followed by the VERATAG® assay. The VERATAG® assay was initiated by the addition of the VERATAG®-conjugated and biotin-conjugated antibody pair followed by washing and incubation with a streptavidin-conjugated photo-sensitizer (ie SA-methylene blue, or SA-MB). The cell line and tumor sections were exposed to light illumination at 670 nm during which the photo-sensitizer bound to the biotin antibody converted dissolved oxygen to a more reactive, singlet state oxygen (O₂) in buffer solution. This occurs via absorption, intersystem crossing and O₂ production.

The O₂ molecules are short-lived (~4 µs in water) and thus have a limited average diffusion distance, *e.g.,* 50% of the O₂ produced will diffuse ~80 nm and <0.1% will diffuse 250 nm before being quenched (Latch, Science, 2006). Consequently, the diffusing 0₂ reacts with the covalent linker between the VERATAG® reporter molecule and the antibody, leading to proximity-based cleavage of the thio-ether bonds and release of VERATAG® reporter molecules bound on the tissue cells *(See* eg, Figure 2A and 2B). Applied to conventional capillary electrophoresis (CE) instruments, the released VERATAG® reporter is separated according to its migration properties and detected as a fluorescence peak in an electropherogram, which can be identified and quantified as the peak area using VERATAG® Informer software. The VERATAG® fluorescent reporter molecule peak area is, therefore, directly proportional to the amount of the target antigen present in the cells. The VERATAG® peak area is initially calculated in RFU. To correct the VERATAG® signal for variable recovery from tissue sections and injection into CE, the peak area (RPA) is calculated relative to that of a known concentration of the internal standard fluorescein.

To identify a proximity pair of Her-2 antibodies suitable for VERATAG® assay development, five antibodies were conjugated with either VERATAG® fluorescent reporter groups or biotin, and ten proximity pairs were tested at 1 ug/mL each on FFPE-prepared human breast tumor cell lines. The performance of each antibody pair was evaluated by their ability to parallel the relative Her-2 protein expression levels, determined by FACS analysis and other independent methods in SK-BR-3 (6x10⁵ per cell), MDA-MB-453 (1.5 x 10⁵ per cell); BT-20 (6x10⁴ per cell); MCF-7 (2 x10⁴ per cell), and MDA-MB-468 cells (negative control; <10⁴ per cell). The Her-2 antibody pair Ab15 and Ab8 generated the greatest dynamic range of signal, consistent with the relative Her-2 expression level quantified by other methods. Representative electropherograms of the VERATAG® signal generated for four well characterized FFPE breast cancer cell lines are shown in Figure 3, along with a parallel Her-2 IHC micrographs utilizing DAB color development. The peak area of the VERATAG® generated from the Her-2 VERATAG® assay parallels IHC signal intensity and is consistent with accepted IHC test categories of Her-2 expression level (ie HercepTest: SK-BR-3 = 3+; MDA-MB-453 = 2+; MCF-7 = 0-1+; MDA-MB-468 = 0).

### Example 10: VERATAG® Antibody and Assay Optimization

Having identified an antibody pair suitable for VERATAG® assay development in the proximity format, relative affinity and specificity were determined for the individual antibodies under non-proximal, direct VERATAG® release conditions, as well as a K_{1/2} and saturating concentrations under proximal conditions. Antibody titrations were performed with VERATAG®conjugated Ab8-Pro11 or Ab15-Pro11 on positive (SKBR-3) and negative (MDA-MB-468) Her-2-expressing FFPE cell lines utilizing a saturating concentration (200uM) of the O₂ sensitizer methylene blue for VERATAG® release. This non-proximal release of Pro11 VERATAG® from increasing concentrations of bound antibody reflects the antibody's relative affinity. The multi-parameter curve fitting result for Ab8-Prol 1 is most consistent with a single binding site of K_{D} = 6-8ug/mL (40-50nM) and similar to the single site binding of Ab 15-Pro 11 with a K_{D} of 2-3ug/mL (12-18nM). The non-specific binding of Ab8-Prol 1 can be estimated from the negative control MDA-MB-468 as <4% percent of the total SK-BR-3 signal, whereas the non-specific binding of Ab15-Pro11 is estimated to be ∼10%.

Optimal Ab8-Pro11 and Ab15-biotin concentrations for the proximity assay of total Her-2 were determined by antibody titrations on FFPE breast cancer cell lines and human breast tumor samples. The concentrations of both antibodies were held equal during the titration from 0.25ug/mL to 8ug/mL. A K₁₁₂ of maximal VERATAG® signal equal to approximately 2ug/mL was observed for both antibodies, and a saturating concentration reached at 3-4ug/mL. In this and other similar titration studies, the optimal signal-to-background ratio of 100-200 is 2-4ug/mL for both Ab8-Prol1 and Ab15-biotin. Additional optimization experiments determined that the concentration of the O₂-sensitizing reagent SA-MB of 2.5ug/mL is saturating under most conditions and the optimal illumination time is 2 hours. Given these results, a concentration of 4ug/mL (26nM) was chosen for both Ab8-Pro l1 and Ab15-biotin, and 2.5ug/mL for SA-MB, for further assay optimization and characterization of performance.

Three Her-2 VERATAG® assay formats were compared at 4ug/mL antibody concentration to identify conditions that result in the best assay performance. These are two proximity formats, consisting of Ab15-biotin plus Ab8-Prol1 and Ab8-biotin plus Ab15-Pro l1, and the non-proximity direct release of VERATAG® from Ab8-Pro l1 in the presence of saturating methylene blue. Although the methylene blue direct release format provides highest overall signal, both proximity assay methods result in lower background, higher signal to background ratio and dynamic range, and tracked most closely with expected receptor number per cell determined by independent methods. The proximity format using Ab15-biotin and Ab8-Prol1 results in the best signal to background ratio and was selected as the final assay format for further study.

The biological sample where the amount of Her-2 and/or Her-2 homodimers is medium was further analyzed for the expression of Her-3 using the methods described above. Thus, the medium Her-2 expressors were further stratified or classified by the level of Her-3 expressors being high or low.

### Example 11: Application of Her-2 and Her-3 classification in patients with metastatic breast cancer

The VERATAG® assay was performed in a cohort of patients with MBC. This cohort (N=103) was derived from the International Serum Her2/neu Study Group (ISHSG) and is called the Lipton cohort. IHC was performed on the patients at a central location - the University of Vienna in Austria - by a single pathologist, where the patients were IHC 3+ or 2+/FISH positive. From the 103 patients, 99 had central FISH measurements, 98 had H2T measurements, and 79 had H3T measurements. Of the 79 patients thus selected, 3 were excluded that were FISH negative with H2T ≥ 1.14. Thus, a final test group of 76 patients were tested.

Five groups were tested as follows:

| | | |
|---|---|---|
| Group 1 | FISH negative | H2T < 1.14 |
| Group 2 | FISH positive | H2T < 1.14 |
| Group 3 | FISH positive | H2T ≥ 1.84 |
| Group 4 | FISH positive | H2T ≥ 1.14, <1.84, H3T high |
| Group 5 | FISH positive | H2T ≥ 1.14, ,1.84, H3T Low |

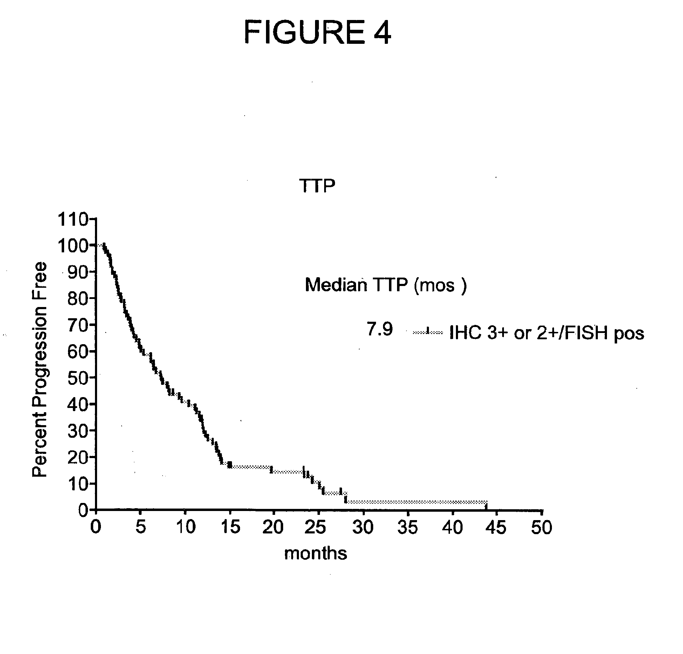

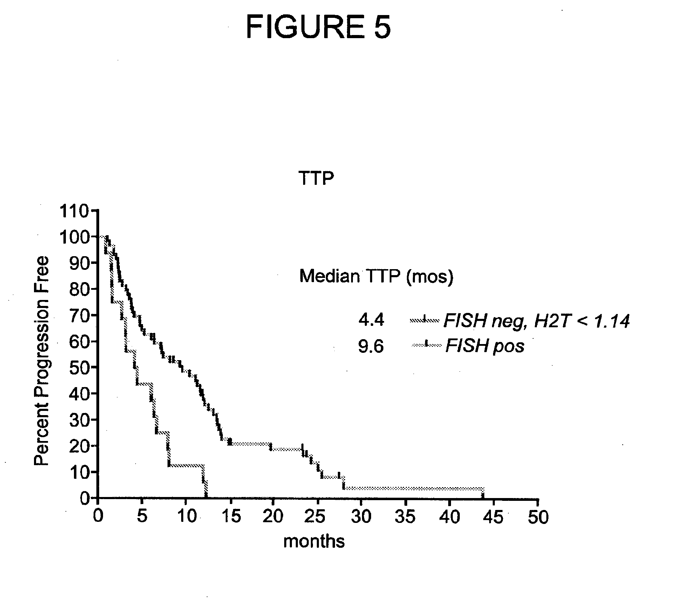

As shown in Figure 4, the total pool of Her-2 positive patients had a median time to progression (TTP) of 7.9 months. The data for patients in Group 1 is shown in Figure 5, and shows a median TTP of 4.4 months.

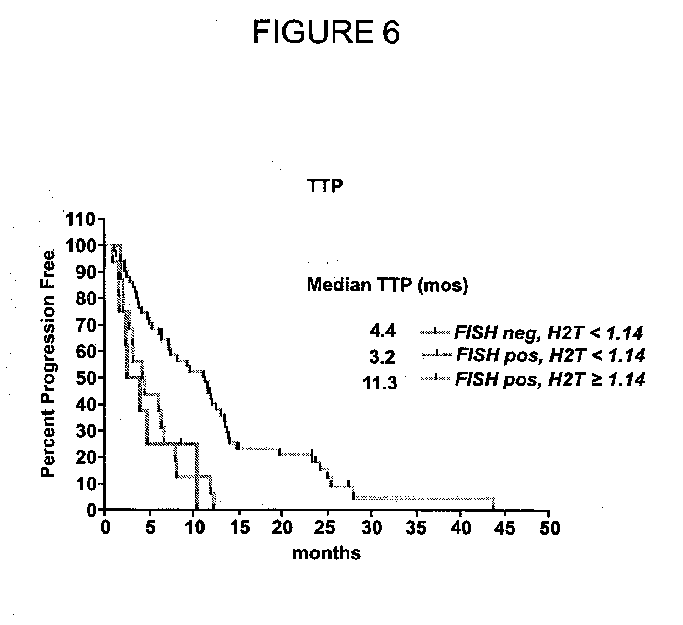

The data for patients in Group 2 that are FISH positive and low HER-2 expressors is shown in Figure 6, and shows a median TTP for FISH negative and H2T <1.14 of 4.4 months, FISH positive and H2T <1.14 of 3.2 months, and FISH positive, and H2T ≥ 1.14 of 11.3 months. Relative to the FISH positive, H2T≥1.14 group, the FISH negative, H2T < 1.14 group (HR=2.7; p=0.0002) and FISH positive, H2T < 1.14 group (HR=2.9; p=0.004) experienced worse outcomes.

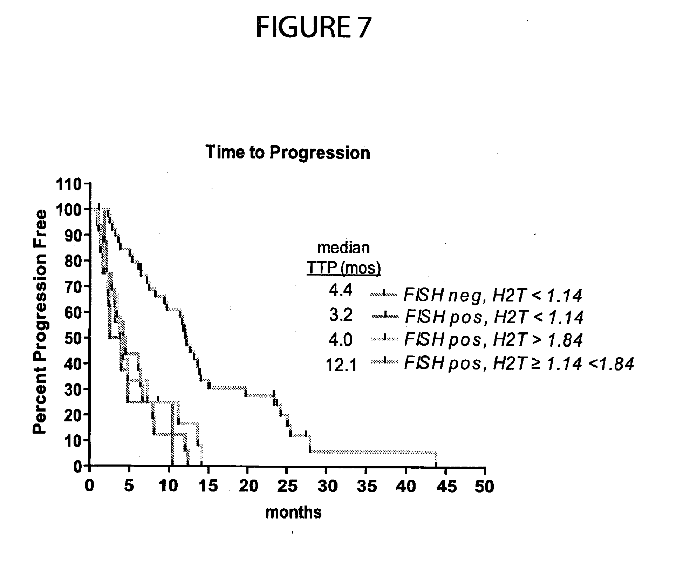

The data for patients in Group 3 that are FISH positive and high HER-2 expressors is shown in Figure 7, and shows a TTP for FISH positive, and H2T ≥ 1.14 < 1.84 of 12.21 months. Thus, this data shows that patients that were FISH positive having moderately high H2T (e.g., patients with H2T ≥ 1.14 and < 1.84) perform better that FISH positive patients with low H2T (e.g., H2T < 1.14) (HR=4.0; p=0.0002), FISH negative patients with low H2T (HR=3.5; p<0.0001) or FISH positive patients with high H2T (e.g., H2T ≥ 1.84) (HR=3.0; p=0.0005).

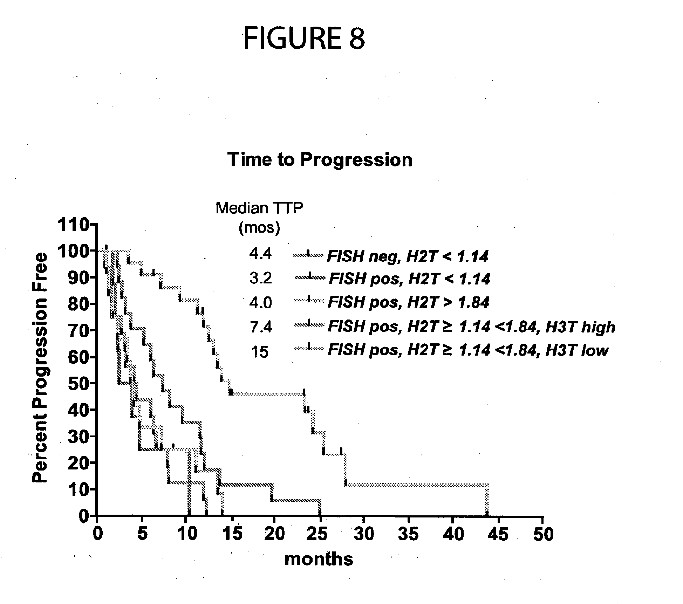

The data for patients in Groups 4 and 5 that are FISH positive and high HER-2 expressors and that are further stratified by either high or low Her-3 expression is shown in Figure 8, and shows a median TTP for FISH positive, and H2T ≥ 1.14, < 1.84 (e.g., moderately high H2T) and high Her-3 expression of 7.4 months and a median TTP for FISH positive, and H2T ≥ 1.14 < 1.84 (e.g., moderately high H2T) and a median TTP for low Her-3 expression of 15.0 months.

For the five groups, the median TTP (months) and hazard ratio relative to FISH negative, H2T < 1.14 is given below:

| | median TTP (months) | Hazard Ratio (p-value) vs. Group 1 | Hazard Ratio (p-value) vs. Group 5 |
|---|---|---|---|
| Group 1 | 4.4 | N/A | 4.9 (<0.0001) |
| Group 2 | 3.2 | 1.1 (0.84) | 5.7 (<0.0001) |
| Group 3 | 4 | 1.3 (0.53) | 4.2 (<0.0001) |
| Group 4 | 7.4 | 0.53 (0.051) | 3.1 (0.0003) |
| Group 5 | 15 | 0.2 (<0.0001) | N/A |

The data thus indicates that a significant number of patients that were treated with trastuzumab based on IHC are FISH-negative and did not respond to treatment. A sub-group of FISH-positive patients that had low Her-2 expression as measured by HERmark did not respond to treatment with trastuzumab while another sub-group of FISH-positive patients that had very high Her-2 expression as measured by HERmark also did not respond to treatment with trastuzumab. The third subgroup of FISH-positive patients that had moderately high (e.g., medium) Her-2 expression as measured by HERmark showed the best response to treatment with trastuzumab.

The third subgroup of FISH-positive patients that had moderately high (e.g., medium) Her-2 expression could be further sub-divided based on the level of Her-3 expression. The data shows that in the third sub-group, patients with high Her-3 expression (Group 4) had a significantly longer TTP (p=0.051) than FISH negative, low Her-2 expressing patients (Group 1) and had about half the risk of progression. In contrast, patients in the third sub-group with low Her-3 expression (Group 5) had the best response of any sub-group, with a 5-fold decrease in risk compared to FISH-negative, low Her-2 expression patients (Group 1) and had significantly better response than intermediate Her-2 and high Her-3 expressors (p=0.0003).

The data thus show that it is possible to determine whether a patient with a Her-2 positive cancer is likely to respond to treatment with a Her2-acting agent and/or for predicting a time course of disease by measuring the amount of Her-2 and/or Her-2 homodimers, wherein if the amount of Her-2 and/or Her-2 homodimers is moderately high (e.g.,medium), then further classifying the patient by measuring the amount of Her-3. Patients that are treated with Her-2 acting agent, and that have medium Her-2 expression but high Her-3 expression will have a longer TTP, while patients that have medium Her-2 expression but low Her-3 expression will respond the best.

### Example 12 Application of Her-2 and Her-3 classification inpatients with breast cancer in the early stage (i.e., adjuvant) setting

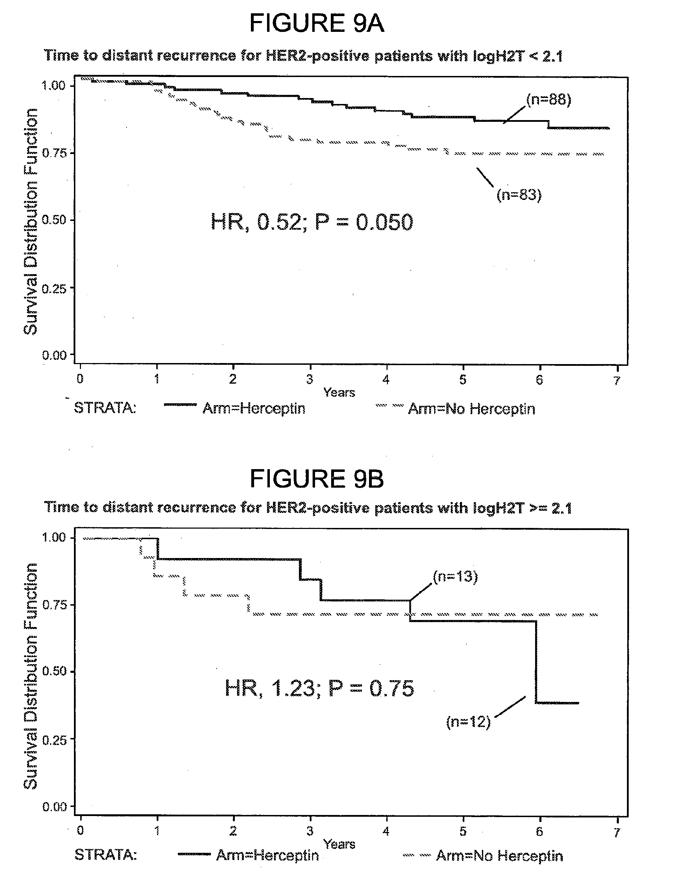

FinHer (Joensuu et al, NEngl JMed 2006, J Clin Oncol 2009) is one of the several prospective randomized clinical trials that show a clinical benefit from trastuzumab added to early stage (i.e., adjuvant) chemotherapy.We investigated the relationship between clinical benefit from trastuzumab and quantitative HER2 protein expression (H2T) as determined by the HERmark assay.Formalin-fixed, paraffin-embedded (FFPE) tissue from 899 invasive breast cancer cases of the FinHer study that had adequate invasive tumor tissues for the HERmark assay were included. 196 of these were HER2-positive by CISH. In this study patients with HER2-positive cancer (n=232) were randomly assigned to receive either trastuzumab administered concomitantly with chemotherapy for 9 weeks or to chemotherapy alone. In this study we focused on the patients with HER2-positive cancer who were randomized to trastuzumab treatment or control. Positional scanning analyses were conducted to identify the optimal cutoff discriminating the very high H2T group. As shown in Figure 9, patients with very high H2T values (log H2T>=2.21; ≥ 125.9 HERmark units) did not benefit from trastuzumab plus chemotherapy treatment relative to controls (HR=1.23, P = 0.75 for TDR, HR=1.05, P = 0.95 for OS), while those with H2T values <125.9 did (HR=0.52, P = 0.05 for TDR, HR=0.48, P =0.1 for OS).

While the preferred embodiment of the invention has been illustrated and described, it will be appreciated that various changes can be made therein without departing from the scope of the invention.

## Claims

1. A method for determining whether a subject with a cancer is likely to respond to treatment with a HER2 acting agent, for predicting a time course of disease, and/or for predicting the probability of a significant event in the time course of the subject's cancer, wherein the significant event is a reduced time between diagnosis with the cancer and at least one of primary diagnosis, progression of the cancer from one stage to a more advanced stage, progression to metastatic disease, relapse, surgery or death, comprising the steps of:
(a) measuring the expression of HER2 in a biological sample from the subject's cancer;
(b) determining whether the biological sample has a low level of HER2, a moderately high level of HER2, or a very high level of HER2, wherein the low level comprises having an amount of HER2 equal to or below a first HER2 threshold level, the moderately high level comprises an amount of HER2 greater than the first HER2 threshold level and less than a second HER2 threshold level, and the very high level comprises an amount of HER2 greater than or equal to the second HER2 threshold level, and wherein the second HER2 threshold level is higher than the first HER2 threshold level;
(c) correlating the amount of HER2 measured in the biological sample to at least one of the likelihood of the subject responding to treatment with a HER2 acting agent, time course of disease, and/or the probability of a significant event in the time course of the subject's cancer;
(d) indicating that the subject is more likely to respond to treatment with a HER2 acting agent, more likely to have a long time course of disease, and/or less likely to have a significant event if the amount of HER2 in the biological sample is moderately high as compared to if the amount of HER2 is low or very high.

2. The method of claim 1, wherein the method further comprises the steps of:
(e) measuring the expression of HER3 in the biological sample;
(f) determining whether the amount of HER3 in the biological sample is low or high, wherein a low level comprises an amount of HER3 below a HER3 threshold level and a high-level comprises an amount of HER3 equal to or above the HER3 threshold level;
(g) correlating the amount of HER3 measured in the biological sample to at least one of the relative likelihood of the subject responding to treatment with a HER2 acting agent, time course of disease, and/or the probability of a significant event in the time course of the subject's cancer; and
(h) indicating that the subject is more likely to respond to treatment with a HER2 acting agent, more likely to have a long time course of disease, and/or less likely to have a significant event if the amount of HER2 in the biological sample is moderately high and the amount of HER3 is low.

3. The method of any one of claims 1 or 2, wherein the HER2 expression comprises HER2 total and/or HER2 homodimers.

4. The method of claims 2 or 3, wherein the HER3 expression comprises HER3 total, HER3 homodimers, or HER3 heterodimers.

5. The method of any one of the preceding claims, wherein the subject's cancer comprises a carcinoma, a lymphoma, a blastoma, a sarcoma, or a leukemia.

6. The method of any one of the preceding claims, wherein the subject's cancer comprises a squamous cell cancer, a lung cancer, a gastrointestinal cancer, a pancreatic cancer, a glioblastoma, a cervical cancer, an ovarian cancer, liver cancer, a bladder cancer, a hepatoma, a breast cancer, a colon cancer, a colorectal cancer, an endometrial carcinoma, a salivary gland carcinoma, a kidney cancer, a prostate cancer, a vulval cancer, a thyroid cancer, a hepatic carcinoma, and/or a head and neck cancer.

7. The method of any one of the preceding claims, wherein the subject's cancer comprises metastatic cancer or primary early stage breast cancer.

8. The method of any one of the preceding claims, wherein the cancer comprises a HER2 positive cancer.

9. The method of any one of the preceding claims, wherein the likelihood to respond is measured with respect to overall survival rate, time to progression, and/or using the RECIST or other response criteria.

10. The method of any one of the preceding claims, wherein the subject is being treated with a HER2 acting agent.

11. The method of any one of the preceding claims, wherein the HER2 acting agent comprises at least one of trastuzumab, lapatinib, AEE-788 or 4D5.

12. The method of any one of the preceding claims, wherein the biological sample comprises a formalin-fixed paraffin-embedded (FFPE) sample.

13. The method of any one of the preceding claims, wherein the amount of HER2 and/or HER3 expression is measured using a VERATAG® assay, a FRET assay, a BRET assay, and/or a biomolecular complementation and proximity ligation assay.

14. The method of any one of the preceding claims, wherein the amount of HER2 expression and/or HER3 expression is measured using an assay capable of measuring and/or quantifying an amount of protein-protein interactions in a sample.

15. The method of any one of the preceding claims, wherein the HER3 expression comprises HER2/HER3 heterodimers.

16. The method of any one of the preceding claims, wherein the amount of HER2 and/or HER3 expression is measured by contacting the biological sample with a binding compound specific for HER2 or HER3, wherein each binding compound has a molecular tag attached thereto by a cleavable linkage, and a cleaving probe having a cleavage-inducing moiety, and detecting whether the molecular tag corresponding to the binding compound specific for HER2 and/or HER3 is released.

## Patentansprüche

1. Verfahren zum Bestimmen, ob eine Person mit einer Krebserkrankung wahrscheinlich auf eine Behandlung mit einem HER2-Wirkstoff anspricht, zum Vorhersagen eines zeitlichen Krankheitsverlaufs und/oder zum Vorhersagen der Wahrscheinlichkeit eines signifikanten Ereignisses innerhalb des zeitlichen Verlaufs der Krebserkrankung der Person, wobei das signifikante Ereignis eine verringerte Zeit zwischen einer Diagnose der Krebserkrankung und mindestens einer Primärdiagnose, eine Progression der Krebserkrankung von einem Stadium zu einem weiter fortgeschrittenen Stadium, eine Progression einer Metastasierung, ein Rückfall, ein chirurgischer Eingriff oder der Tod ist, wobei das Verfahren die folgenden Schritte umfasst:
(a) Messen der Expression von HER2 in einer biologischen Probe von der Krebserkrankung der Person;
(b) Bestimmen, ob die biologische Probe einen niedrigen HER2-Spiegel, einen mäßig hohen HER2-Spiegel oder einen sehr hohen HER2-Spiegel aufweist, wobei der niedrige Spiegel das Aufweisen einer HER2-Menge umfasst, die kleiner gleich einem ersten HER2-Grenzspiegel ist, der mäßig hohe Spiegel eine HER2-Menge umfasst, die größer als der erste HER2-Grenzspiegel und kleiner als ein zweiter HER2-Grenzspiegel ist, und der sehr hohe Spiegel eine HER2-Menge umfasst, die größer gleich dem zweiten HER2-Grenzspiegel ist, und wobei der zweite HER2-Grenzspiegel höher als der erste HER2-Grenzspiegel ist;
(c) Korrelieren der in der biologischen Probe gemessenen HER2-Menge mit der Wahrscheinlichkeit des Ansprechens der Person auf eine Behandlung mit einem HER2-Wirkstoff, dem zeitlichen Krankheitsverlauf und/oder der Wahrscheinlichkeit eines signifikanten Ereignisses innerhalb des zeitlichen Verlaufs der Krebserkrankung der Person;
(d) Anzeigen, dass es wahrscheinlicher ist, dass die Person auf eine Behandlung mit einem HER2-Wirkstoff anspricht, es wahrscheinlicher ist, dass die Person einen langen zeitlichen Krankheitsverlauf hat, und/oder es weniger wahrscheinlich ist, dass die Person ein signifikantes Ereignis hat, wenn die HER2-Menge in der biologischen Probe mäßig hoch ist, verglichen damit, wenn die HER2-Menge niedrig oder sehr hoch ist.

2. Verfahren nach Anspruch 1, wobei das Verfahren weiterhin die folgenden Schritte umfasst:
(e) Messen der Expression von HER3 in der biologischen Probe;
(f) Bestimmen, ob die HER3-Menge in der biologischen Probe niedrig oder hoch ist, wobei ein niedriger Spiegel eine HER3-Menge umfasst, die unter einem HER3-Grenzspiegel liegt, und ein hoher Spiegel eine HER3-Menge umfasst, die größer gleich dem HER3-Grenzspiegel ist;
(g) Korrelieren der in der biologischen Probe gemessenen HER3-Menge mit der relativen Wahrscheinlichkeit des Ansprechens der Person auf eine Behandlung mit einem HER2-Wirkstoff, dem zeitlichen Krankheitsverlauf und/oder der Wahrscheinlichkeit eines signifikanten Ereignisses innerhalb des zeitlichen Verlaufs der Krebserkrankung der Person und
(h) Anzeigen, dass es wahrscheinlicher ist, dass die Person auf eine Behandlung mit einem HER2-Wirkstoff anspricht, es wahrscheinlicher ist, dass die Person einen langen zeitlichen Krankheitsverlauf hat, und/oder es weniger wahrscheinlich ist, dass die Person ein signifikantes Ereignis hat, wenn die HER2-Menge in der biologischen Probe mäßig hoch ist und die HER3-Menge niedrig ist.

3. Verfahren nach einem der Ansprüche 1 oder 2, wobei die HER2-Expression HER2 insgesamt und/oder HER2-Homodimere umfasst.

4. Verfahren nach den Ansprüchen 2 oder 3, wobei die HER3-Expression HER3 insgesamt, HER3-Homodimere oder HER3-Heterodimere umfasst.

5. Verfahren nach einem der vorhergehenden Ansprüche, wobei die Krebserkrankung der Person ein Karzinom, ein Lymphom, ein Blastom, ein Sarkom oder eine Leukämie umfasst.

6. Verfahren nach einem der vorhergehenden Ansprüche, wobei die Krebserkrankung der Person ein Plattenepithelkarzinom, eine Lungenkrebserkrankung, eine Magen-Darm-Krebserkrankung, eine Bauchspeicheldrüsenkrebserkrankung, ein Glioblastom, eine Gebärmutterhalskrebserkrankung, eine Eierstockkrebserkrankung, Leberkrebs, eine Blasenkrebserkrankung, ein Hepatom, eine Brustkrebserkrankung, eine Dickdarmkrebserkrankung, ein kolorektales Karzinom, ein Endometriumkarzinom, ein Speicheldrüsenkarzinom, eine Nierenkrebserkrankung, eine Prostatakrebserkrankung, eine Vulvakrebserkrankung, eine Schilddrüsenkrebserkrankung, ein Leberkarzinom und/oder eine Kopf- und Hals-Krebserkrankung umfasst.

7. Verfahren nach einem der vorhergehenden Ansprüche, wobei die Krebserkrankung der Person ein metastasierendes Karzinom oder primären Brustkrebs im Frühstadium umfasst.

8. Verfahren nach einem der vorhergehenden Ansprüche, wobei die Krebserkrankung eine HER2-positive Krebserkrankung umfasst.

9. Verfahren nach einem der vorhergehenden Ansprüche, wobei die Wahrscheinlichkeit des Ansprechens in Bezug auf die Gesamtüberlebensrate, die Zeit bis zur Progression und/oder die Verwendung der RECIST- oder anderer Ansprechkriterien gemessen wird.

10. Verfahren nach einem der vorhergehenden Ansprüche, wobei die Person mit einem HER2-Wirkstoff behandelt wird.

11. Verfahren nach einem der vorhergehenden Ansprüche, wobei der HER2-Wirkstoff Trastuzumab, Lapatinib, AEE-788 und/oder 4D5 umfasst.

12. Verfahren nach einem der vorhergehenden Ansprüche, wobei die biologische Probe eine mit Formalin fixierte, in Paraffin eingebettete Probe (FFPE-Probe) umfasst.

13. Verfahren nach einem der vorhergehenden Ansprüche, wobei der Umfang der HER2- und/oder der HER3-Expression unter Verwendung eines VERATAG®-Assays, eines FRET-Assays, eines BRET-Assays und/oder eines biomolekularen Komplementierungs- und Proximity-Ligation-Assays gemessen wird.

14. Verfahren nach einem der vorhergehenden Ansprüche, wobei der Umfang der HER2-Expression und/oder der HER3-Expression unter Verwendung eines Assays gemessen wird, der einen Umfang von Protein-Protein-Interaktionen in einer Probe gemessen und/oder quantifizieren kann.

15. Verfahren nach einem der vorhergehenden Ansprüche, wobei die HER3-Expression HER2/HER3-Heterodimere umfasst.

16. Verfahren nach einem der vorhergehenden Ansprüche, wobei der Umfang der HER2- und/oder der HER3-Expression gemessen wird, indem die biologische Probe mit einer Bindungsverbindung, die für HER2 oder HER3 spezifisch ist, in Kontakt gebracht wird, wobei jede Bindungsverbindung einen molekularen Marker, der durch eine spaltbare Verknüpfung daran angelagert ist, und eine Spaltungssonde mit einer die Spaltung induzierenden Einheit aufweist, und nachgewiesen wird, ob der molekulare Marker, der der Bindungsverbindung, die für HER2 und/oder HER3 spezifisch ist, entspricht, freigesetzt wird.

## Revendications

1. Procédé consistant à déterminer si un sujet atteint d'un cancer est enclin à répondre à un traitement par un agent ciblant HER2, à prévoir l'évolution temporelle d'une maladie, et/ou à prévoir la probabilité d'un événement significatif pendant l'évolution temporelle du cancer du patient, l'événement significatif étant un délai réduit entre le diagnostic du cancer et au moins un événement parmi les événements suivants : diagnostic primaire, progression du cancer d'un stade à un stade plus avancé, progression à un état métastatique de la maladie, rechute, chirurgie ou décès, ledit procédé comprenant les étapes consistant à :
(a) mesurer l'expression de HER2 dans un échantillon biologique du cancer du sujet ;
(b) déterminer si l'échantillon biologique a un niveau faible de HER2, un niveau modérément élevé de HER2 ou un niveau très élevé de HER2, le niveau faible comprenant une quantité de HER2 inférieure ou égale à un premier niveau de seuil de HER2, le niveau modérément élevé comprenant une quantité de HER2 supérieure au premier niveau de seuil de HER2 et inférieure à un deuxième niveau de seuil de HER2, et le niveau très élevé comprenant une quantité de HER2 supérieure ou égale au deuxième niveau de seuil de HER2, et le deuxième niveau de seuil de HER2 étant supérieur au premier niveau de seuil de HER2 ;
(c) corréler la quantité de HER2 mesurée dans l'échantillon biologique à la probabilité que le sujet répondre à un traitement par un agent ciblant HER2, à l'évolution temporelle de la maladie, et/ou à la probabilité d'un événement significatif pendant l'évolution temporelle du cancer du sujet ;
(d) indiquer que le sujet est plus enclin à répondre à un traitement par un agent ciblant HER2, plus enclin à avoir une longue évolution temporelle de la maladie, et/ou moins enclin à faire l'objet d'un événement significatif si la quantité de HER2 dans l'échantillon biologique est modérément élevée comparée à si la quantité de HER2 est faible ou très élevée.

2. Procédé selon la revendication 1, le procédé comprenant en outre les étapes consistant à :
(e) mesurer l'expression de HER3 dans l'échantillon biologique ;
(f) déterminer si la quantité de HER3 dans l'échantillon biologique est faible ou élevée, un niveau faible comprenant une quantité de HER3 inférieure à un niveau de seuil de HER3, et un niveau élevé comprenant une quantité de HER3 supérieure ou égale au niveau de seuil de HER3 ;
(g) corréler la quantité de HER3 mesurée dans l'échantillon biologique à la probabilité relative que le sujet répondre à un traitement par un agent ciblant HER2, à l'évolution temporelle de la maladie, et/ou à la probabilité d'un événement significatif pendant l'évolution temporelle du cancer du sujet ; et
(h) indiquer que le sujet est plus enclin à répondre à un traitement par un agent ciblant HER2, plus enclin à avoir une longue évolution temporelle de la maladie, et/ou moins enclin à faire l'objet d'un événement significatif si la quantité de HER2 dans l'échantillon biologique est modérément élevée comparée et la quantité de HER3 est faible.

3. Procédé selon l'une quelconque des revendications 1 et 2, dans lequel l'expression de HER2 comprend HER2 totale et/ou des homodimères HER2.

4. Procédé selon la revendication 2 ou 3, dans lequel l'expression de HER3 comprend HER3 totale, des homodimères HER3 ou des hétérodimères HER3.

5. Procédé selon l'une quelconque des revendications précédentes, dans lequel le cancer du patient consiste en un carcinome, un lymphome, un blastome, un sarcome ou une leucémie.

6. Procédé selon l'une quelconque des revendications précédentes, dans lequel le cancer du sujet consiste en un cancer à cellules squameuses, un cancer du poumon, un cancer gastro-intestinal, un cancer du pancréas, un glioblastome, un cancer cervical, un cancer de l'ovaire, un cancer du foie, un cancer de la vessie, un hépatome, un cancer du sein, un cancer du côlon, un cancer colorectal, un carcinome de l'endomètre, un carcinome des glandes salivaires, un cancer du rein, un cancer de la prostate, un cancer de la vulve, un cancer de la glande thyroïde, un carcinome hépatique et/ou un cancer de la tête et du cou.

7. Procédé selon l'une quelconque des revendications précédentes, dans lequel le cancer du patient consiste en un cancer métastatique ou un cancer du sein à un stade précoce.

8. Procédé selon l'une quelconque des revendications précédentes, dans lequel le cancer consiste en un cancer HER2-positif.

9. Procédé selon l'une quelconque des revendications précédentes, dans lequel la probabilité de répondre est mesurée par rapport à un taux de survie globale, au délai avant progression et/ou à l'utilisation des critères RECIST ou d'autres critères de réponse.

10. Procédé selon l'une quelconque des revendications précédentes, dans lequel le sujet est traité par un agent ciblant HER2.

11. Procédé selon l'une quelconque des revendications précédentes, dans lequel l'agent ciblant HER2 consiste au moins un agent parmi le trastuzumab, le lapatinib, AEE-788 ou 4D5.

12. Procédé selon l'une quelconque des revendications précédentes, dans lequel l'échantillon biologique consiste en un échantillon fixé au formol et enrobé à la paraffine (FFPE).

13. Procédé selon l'une quelconque des revendications précédentes, dans lequel la quantité d'expression de HER2 et/ou de HER3 est mesurée au moyen d'un essai VERATAG®, d'un essai FRET, d'un essai BRET et/ou d'un essai de complémentation biomoléculaire et de ligature de proximité.

14. Procédé selon l'une quelconque des revendications précédentes, dans lequel la quantité d'expression de HER2 et/ou d'expression de HER3 est mesurée au moyen d'un essai capable de mesurer et/ou de quantifier une quantité d'interactions protéine-protéine dans un échantillon.

15. Procédé selon l'une quelconque des revendications précédentes, dans lequel l'expression de HER3 comprend des hétérodimères HER2/HER3.

16. Procédé selon l'une quelconque des revendications précédentes, dans lequel la quantité d'expression de HER2 et/ou de HER3 est mesurée en mettant en contact l'échantillon biologique avec un composé de liaison spécifique de HER2 ou HER3, chaque composé de liaison comportant une étiquette moléculaire y étant liée par une liaison clivable, et une sonde de clivage comportant un fragment induisant le clivage, et en détectant si l'étiquette moléculaire correspondant au composé de liaison spécifique de HER2 et/ou HER3 est libérée.
